# EUROPÄISCHE PATENTSCHRIFT

(11) **EP 3 272 733 B1**
(45) Veröffentlichungstag und Bekanntmachung des Hinweises auf die Patenterteilung: **02.01.2019**
(21) Anmeldenummer: 16180058.6
(22) Anmeldetag: 19.07.2016
(51) Int. Cl.: C07C 67/38, C07C 69/24

(54) **VERFAHREN ZUR ALKOXYCARBONYLIERUNG VON OLEFINEN IN EINEM MEDIUM MIT GERINGER BRØNSTEDSÄURENKONZENTRATION**
METHOD FOR THE ALCOXYCARBONYLATION OF OLEFINS IN A MEDIUM WITH LOW BRØNSTED ACID CONCENTRATION
PROCEDE D'ALKOXY-CARBONYLATION D'OLEFINES DANS UN MILIEU AYANT UNE CONCENTRATION REDUITE EN ACIDE DE BRONSTED

(43) Veröffentlichungstag der Anmeldung: 24.01.2018
(73) Patentinhaber: Evonik Degussa GmbH, 45128 Essen (DE)
(72) Erfinder: FANG, Xiangjie, Changning District, Shanghai (CN); DONG, Kaiwu, 236800 Bo Zhou (CN); NEUMANN, Helfreid, 18055 Rostock (DE); JACKSTELL, Ralf, 27478 Cuxhaven Altenwalde (DE); BELLER, Matthias, 18211 Ostseebad Nienhagen (DE); FRIDAG, Dirk, 45721 Haltern am See (DE); HESS, Dieter, 45770 Marl (DE); DYBALLA, Katrin Marie, 45657 Recklinghausen (DE); GEILEN, Frank, 45721 Haltern am See (DE); FRANKE, Robert, 45772 Marl (DE)

(56) Entgegenhaltungen:
- FANG X, LI H, JACKSTELL R, BELLER M: "Palladium-Catalyzed Alkoxycarbonylation of Conjugated Dienes under Acid-Free Conditions: Atom-Economic Synthesis of beta,gamma-Unsaturated Esters", ANGEW. CHEM. INT. ED., Bd. 53, Nr. 34, 18. August 2014 (2014-08-18), Seiten 9030-9034, XP055330056, DOI: 10.1002/anie.201404563
- LAILAI WANG ET AL: "One-Step Synthesis of Chiral Dimethyl 2-Oxo-3-phenyl-glutarate in the Asymmetric Triple-carbonylation of Styrene", CHINESE JOURNAL OF CATALYSIS / DALIAN INSTITUTE OF CHEMICAL PHYSICS, Bd. 32, Nr. 6-8, 1. Januar 2011 (2011-01-01), Seiten 1143-1148, XP055332410, AMSTERDAM, NL ISSN: 1872-2067, DOI: 10.1016/S1872-2067(10)60253-7
- REIKO JENNERJAHN ET AL: "Palladium-Catalyzed Isomerization and Hydroformylation of Olefins", CHEMISTRY - A EUROPEAN JOURNAL, Bd. 15, Nr. 26, 22. Juni 2009 (2009-06-22) , Seiten 6383-6388, XP055015624, ISSN: 0947-6539, DOI: 10.1002/chem.200900700

## Beschreibung

Die vorliegende Erfindung betrifft ein Verfahren zur Alkoxycarbonylierung von Olefinen, wobei die Alkoxycarbonylierung in einem Medium mit geringer Brønstedsäurenkonzentration stattfindet.

Die Alkoxycarbonylierung von Alkenen ist ein Prozess mit steigender Bedeutung. Unter einer Alkoxycarbonylierung versteht man die Umsetzung von ungesättigten Verbindungen, wie Olefinen, mit Kohlenmonoxid und Alkoholen in Gegenwart eines Metalls bzw. eines Metallkomplexes und eines Liganden zu den entsprechenden Estern:

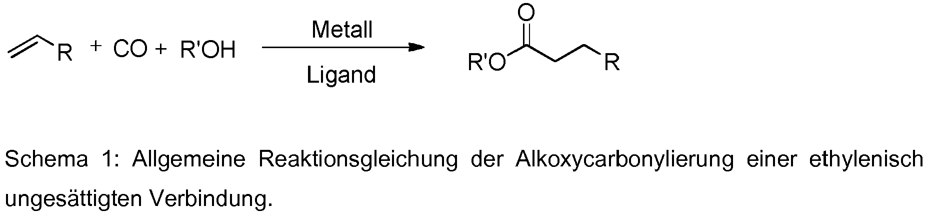

Unter den Alkoxycarbonylierungsreaktionen ist die Ethen-Methoxycarbonylierung zu 3-Methylpropionat von Bedeutung als Zwischenstufe für die Herstellung von Methylmethacrylat (S. G. Khokarale, E. J. García-Suárez, J. Xiong, U. V. Mentzel, R. Fehrmann, A. Riisager, Catalysis Communications 2014, 44, 73-75). Die Ethen-Methoxycarbonylierung wird in Methanol als Lösemittel bei milden Bedingungen mit einem durch Phosphinliganden modifizierten Palladiumkatalysator durchgeführt. Ein sehr gutes katalytischen System wurde von Lucite - jetzt Mitsubishi Rayon - entwickelt und verwendet einen Liganden auf Basis von 1,2-Bis(di-tert-butylphosphinomethyl)benzol (DTBPMB) (W. Clegg, G. R. Eastham, M. R. J. Elsegood, R. P. Tooze, X. L. Wang, K. Whiston, Chem. Commun 1999, 1877-1878).

Die Alkoxycarbonylierung wird üblicherweise unter Zugabe von starken Brønstedsäuren durchgeführt, beispielsweise para-Toluolsulfonsäure, Methansulfonsäure oder Schwefelsäure. Dies ist allerdings von Nachteil für den industriellen Einsatz der Alkoxycarbonylierung, da es mit einer erhöhten Korrosion der Reaktoren und sonstigen Anlagen einhergeht.

Ein bekanntes säurefreies Verfahren beruht auf der Verwendung von 4,5-Bis(diphenylphosphino)-9,9-dimethylxanthene (Xantphos) als zweizähnigem Liganden (Fang, X., Li, H., Jackstell, R. und Beller, M., 2014, Palladium-Catalyzed Alkoxycarbonylation of Conjugated Dienes under Acid-Free Conditions: Atom-Economic Synthesis of β,γ-Unsaturated Esters. Angew. Chem. Int. Ed., 53: 9030-9034). Allerdings wurde für eine Anzahl weiterer zweizähniger Liganden gefunden, dass sie sich nicht für die säurefreie Alkoxycarbonylierung eignen (Fang, X. *et al.,* aaO).

Somit besteht die Aufgabe der vorliegenden Erfindung in der Bereitstellung eines Verfahrens zur Alkoxycarbonylierung von Olefinen, bei dem die Zugabe an Brønstedsäuren gering gehalten werden kann und gute Ausbeuten erzielt werden können.

Diese Aufgabe wird gelöst durch ein Verfahren umfassend die Verfahrensschritte:
a) Vorlegen einer ethylenisch ungesättigten Verbindung;
b) Zugabe eines Ligand-Metall-Komplexes umfassend Pd und einen zweizähnigen Phosphinliganden,
   oder Zugabe eines zweizähnigen Phosphinliganden und einer Verbindung, welche Pd umfasst;
c) Zugabe eines Alkohols;
d) Zuführen von CO;
e) Erwärmen des Reaktionsgemisches, wobei die ethylenisch ungesättigte Verbindung zu einem Ester umgesetzt wird,
wobei dem Reaktionsgemisch weniger als 0,1 mol-%, bezogen auf die Stoffmenge der ethylenisch ungesättigten Verbindung, Brønstedsäuren mit einer Säurestärke von pKs ≤ 3 zugesetzt werden,
dadurch gekennzeichnet, dass der Phosphinligand an wenigstens einem Phosphoratom mit wenigstens einem Heteroarylrest substituiert ist.

Hierbei können die Verfahrensschritte a), b), c) und d) in beliebiger Reihenfolge erfolgen. Üblicherweise erfolgt die Zugabe von CO jedoch, nachdem die Reaktionspartner in den Schritten a) bis c) vorgelegt wurden. Die Schritte d) und e) können gleichzeitig oder nacheinander erfolgen. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Durch die erfindungsgemäße Verwendung eines zweizähnigen Phosphinliganden, der an wenigstens einem Phosphoratom mit wenigstens einem Heteroarylrest substituiert ist, ist es möglich die Alkoxycarbonylierung von ethylenisch ungesättigten Verbindungen ohne Zusatz von starken Brønstedsäuren mit einer Säurestärke von pKₛ ≤ 3, bzw. unter Zusatz von maximal 0,1 mol-%, bezogen auf die Stoffmenge der ethylenisch ungesättigten Verbindung, durchzuführen, ohne dass dies mit Ausbeuteverlusten einhergeht. Dadurch werden insbesondere Korrosionsprobleme vermieden bzw. es müssen keine teuren, säurebeständigen Stähle in der Großanlage eingesetzt werden.

Vorzugsweise liegt die Menge an zugesetzter Brønstedsäure mit einer Säurestärke von pKₛ ≤ 3 im Reaktionsgemisch bei weniger als 0,01 mol-%, bevorzugt weniger als 0,001 mol-%, besonders bevorzugt weniger als 0,0001 mol-%, am meisten bevorzugt 0 mol-%, bezogen auf die Stoffmenge der ethylenisch ungesättigten Verbindung. Der Anteil der Brønstedsäure wird dabei auf Grundlage der gesamten Stoffmenge aller zugegebenen Brønstedsäuren mit einer Säurestärke von pKₛ ≤ 3 berechnet. Als Stoffmenge der ethylenisch ungesättigten Verbindung wird die gesamte Stoffmenge der vorgelegten ethylenisch ungesättigten Verbindung zugrunde gelegt.

Im Sinne dieser Erfindung bezeichnet der Begriff "Brønstedsäuren" Verbindungen, die Protonen abgeben können (Protonendonatoren). Dieser Begriff umfasst somit nicht LewisSäuren (Elektronenpaarakzeptoren), die keine Protonen abgeben können, wie beispielsweise die Pd-Verbindung PdCl₂.

Der Begriff "säurefrei" in Zusammenhang mit dieser Erfindung bezieht sich somit auf den Umstand, dass dem Reaktionsgemisch nicht aktiv Brønstedsäuren zugesetzt werden. Eine Bildung von Brønstedsäuren durch den Reaktionsverlauf ist hierdurch nicht gänzlich ausgeschlossen.

Die angegebene Säurestärke von pKₛ bezieht sich auf den bei Normalbedingungen (25°C, 1,01325 bar) bestimmten pKₛ-Wert. Bei einer mehrprotonigen Säure bezieht sich die Säurestärke pKₛ im Rahmen dieser Erfindung auf den pKₛ-Wert des ersten Protolyseschrittes.

Das erfindungsgemäße Verfahren wird vorzugsweise auch ohne Zusatz einer weniger starken Brønstedsäure mit pKs ≤ 5, bevorzugt pKₛ ≤ 6, durchgeführt. Vorzugsweise beträgt somit der Anteil an Brønstedsäure mit einer Säurestärke von pKₛ ≤ 5, bevorzugt pKₛ ≤ 6, im Reaktionsgemisch weniger als 0,1 mol-%, bevorzugt weniger als 0,01 mol-%, weiterhin bevorzugt weniger als 0,001 mol-%, besonders bevorzugt weniger als 0,0001 mol-%, am meisten bevorzugt 0 mol-%, bezogen auf die Stoffmenge der ethylenisch ungesättigten Verbindung.

Zweizähnige Phosphinliganden im Sinne dieser Erfindung sind Liganden, die zwei Phosphingruppen umfassen, wobei die Phosphoratome beider Phosphingruppen zusammen ein Palladiumatom koordinieren können. Es hat sich gezeigt, dass dadurch, dass wenigstens ein Phosphoratom mit wenigstens einer Heteroarylgruppe substituiert ist, eine hohe Ausbeute bei der säurefreien Alkoxycarbonylierung erzielt werden kann.

Vorzugsweise handelt es sich bei der Heteroarylgruppe um eine -(C₃-C₂₀)-Heteroarylgruppe.

Geeignete Heteroarylgruppen sind beispielsweise Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl.

Besonders bevorzugt sind Heteroarylgruppen mit fünf bis zehn Ringatomen, vorzugsweise fünf oder sechs Ringatomen.

Die genannten Heteroarylgruppen können dabei mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl,-CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]2, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl,-COOH, -OH, -SO₃H, -NH₂, Halogen substituiert sein.

Bevorzugt sind dabei Substituenten ausgewählt aus -(C₁-C₁₂)-Alkyl, -(C₃-C₁₂)-Cycloalkyl,-O-(C₁-C₁₂)-Alkyl, -O-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl.

Besonders bevorzugt sind die Phosphoratome beider Phosphingruppen mit wenigstens einer Heteroarylgruppe substituiert.

Der zweizähnige Phosphinligand ist ausgewählt aus einer Verbindung nach einer der Formeln (**I**) und (**II**) 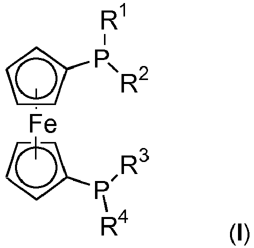 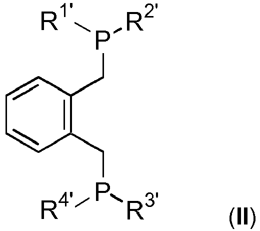 wobei
R¹, R², R³, R⁴, R^{1'}, R^{2'}, R^{3'}, R^{4'} jeweils unabhängig voneinander ausgewählt sind aus -(C₁-C₁₂)-Alkyl, -(C₃-C₁₂)-Cycloalkyl, -(C₃-C₁₂)-Heterocycloalkyl, -(C₆-C₂₀)-Aryl, -(C₃-C₂₀)-Heteroaryl;
mindestens einer der Reste R¹, R², R³, R⁴ bzw. mindestens einer der Reste R^{1'}, R^{2'}, R^{3'}, R^{4'} für einen -(C₃-C₂₀)-Heteroarylrest steht;
und
R¹, R², R³, R⁴, R^{1'}, R^{2'}, R^{3'}, R^{4'}, falls diese für -(C₁-C₁₂)-Alkyl, -(C₃-C₁₂)-Cycloalkyl, -(C₃-C₁₂)-Heterocycloalkyl, -(C₆-C₂₀)-Aryl, oder -(C₃-C₂₀)-Heteroaryl stehen,
jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl,-COO-(C₁-C₁₂)-Alkyl, -COO-(C₃-C₁₂)-Cycloalkyl, -CONH-(C₁-C₁₂)-Alkyl, -CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, n-Butyl, *iso*-Butyl, *sec*-Butyl, *tert*-Butyl, *n*-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, *n*-Heptyl, 2-Heptyl, 3-Heptyl, 2-Ethylpentyl, 1-Propylbutyl, *n*-Octyl, 2-Ethylhexyl, 2-Propylheptyl, Nonyl, Decyl.

Die Erläuterungen zum Begriff (C₁-C₁₂)-Alkyl gelten insbesondere auch für die Alkylgruppen in -O-(C₁-C₁₂)-Alkyl, -S-(C₁-C₁₂)-Alkyl, -COO-(C₁-C₁₂)-Alkyl, -CONH-(C₁-C₁₂)-Alkyl, -CO-(C₁-C₁₂)-Alkyl und -N-[(C₁-C₁₂)-Alkyl]₂.

Der Begriff (C₃-C₁₂)-Cycloalkyl umfasst mono-, bi- oder tricyclische Kohlenwasserstoffgruppen mit 3 bis 12 Kohlenstoffatomen. Vorzugsweise handelt es sich dabei um (C₅-C₁₂)-Cycloalkyl.

Die (C₃-C₁₂)-Cycloalkylgruppen weisen vorzugsweise 3 bis 8, besonders bevorzugt 5 oder 6, Ringatome auf.

Geeignete (C₃-C₁₂)-Cycloalkylgruppen sind insbesondere Cyclopropyl, Cyclobutyl, Cyclopentyl, Cyclohexyl, Cycloheptyl, Cyclooctyl, Cyclododecyl, Cyclopentadecyl, Norbonyl, Adamantyl.

Die Erläuterungen zum Begriff (C₃-C₁₂)-Cycloalkyl gelten insbesondere auch für die Cycloalkylgruppen in -O-(C₃-C₁₂)-Cycloalkyl, -S-(C₃-C₁₂)-Cycloalkyl, -COO-(C₃-C₁₂)-Cycloalkyl, -CONH-(C₃-C₁₂)-Cycloalkyl, -CO-(C₃-C₁₂)-Cycloalkyl.

Der Begriff (C₃-C₁₂)-Heterocycloalkyl umfasst nichtaromatische, gesättigte oder teilweise ungesättigte cycloaliphatische Gruppen mit 3 bis 12 Kohlenstoffatomen, wobei eins oder mehrere der Ringkohlenstoffatome durch Heteroatome ersetzt sind. Die (C₃-C₁₂)-Heterocycloalkylgruppen weisen vorzugsweise 3 bis 8, besonders bevorzugt 5 oder 6, Ringatome auf und sind ggf. mit aliphatischen Seitenketten substituiert. In den Heterocycloalkylgruppen sind im Unterschied zu den Cycloalkylgruppen eins oder mehrere der Ringkohlenstoffatome durch Heteroatome oder heteroatomhaltige Gruppen ersetzt. Die Heteroatome oder die heteroatomhaltigen Gruppen sind vorzugsweise ausgewählt unter O, S, N, N(=O), C(=O), S(=O). Eine (C₃-C₁₂)-Heterocycloalkylgruppe im Sinne dieser Erfindung ist somit auch Ethylenoxid.

Geeignete (C₃-C₁₂)-Heterocycloalkylgruppen sind insbesondere Tetrahydrothiophenyl, Tetrahydrofuryl, Tetrahydropyranyl und Dioxanyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

Der Begriff (C₃-C₂₀)-Heteroaryl umfasst mono- oder polycyclische aromatische Kohlenwasserstoffreste mit 3 bis 20 Kohlenstoffatomen, wobei eins oder mehrere der Kohlenstoffatome durch Heteroatome ersetzt sind. Bevorzugte Heteroatome sind N, O und S. Die (C₃-C₂₀)-Heteroarylgruppen weisen 3 bis 20, bevorzugt 6 bis 14, besonders bevorzugt 6 bis 10 Ringatome auf. Somit ist beispielsweise Pyridyl im Rahmen dieser Erfindung ein C₆-Heteroarylrest, Furyl ist ein C₅-Heteroarylrest.

Geeignete (C₃-C₂₀)-Heteroarylgruppen sind insbesondere Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl.

Der Begriff Halogen umfasst insbesondere Fluor, Chlor, Brom und lod. Besonders bevorzugt sind Fluor und Chlor.

In einer Ausführungsform können die Reste R¹, R², R³, R⁴, R^{1'}, R^{2'}, R^{3'}, R^{4'}, falls sie für -(C₁-C₁₂)-Alkyl, , -(C₃-C₁₂)-Cycloalkyl, -(C₃-C₁₂)-Heterocycloalkyl, -(C₆-C₂₀)-Aryl, oder -(C₃-C₂₀)-Heteroaryl stehen, jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein.

In einer Ausführungsform können die Reste R¹, R², R³, R⁴, R^{1'}, R^{2'}, R^{3'}, R^{4'}, falls sie für -(C₁-C₁₂)-Alkyl, -(C₃-C₁₂)-Cycloalkyl, -(C₃-C₁₂)-Heterocycloalkyl, -(C₆-C₂₀)-Aryl, oder -(C₃-C₂₀)-Heteroaryl stehen, jeweils unabhängig voneinander mit einem oder mehreren Substituenten ausgewählt aus -(C₁-C₁₂)-Alkyl, -(C₃-C₁₂)-Cycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, substituiert sein.

In einer Ausführungsform können die Reste R¹, R², R³, R⁴, R^{1'}, R^{2'}, R^{3'}, R^{4'}, falls sie für -(C₁-C₁₂)-Alkyl, -(C₃-C₁₂)-Cycloalkyl, -(C₃-C₁₂)-Heterocycloalkyl, -(C₆-C₂₀)-Aryl oder -(C₃-C₂₀)-Heteroaryl stehen, jeweils unabhängig voneinander mit einem oder mehreren Substituenten ausgewählt aus -(C₁-C₁₂)-Alkyl, -O-(C₁-C₁₂)-Alkyl-(C₆-C₂₀)-Aryl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl substituiert sein.

In einer Ausführungsform können die Reste R¹, R², R³, R⁴, R^{1'}, R^{2'}, R^{3'}, R^{4'}, falls sie für -(C₁-C₁₂)-Alkyl, -(C₃-C₁₂)-Cycloalkyl, -(C₃-C₁₂)-Heterocycloalkyl, -(C₆-C₂₀)-Aryl, oder -(C₃-C₂₀)-Heteroaryl stehen, jeweils unabhängig voneinander mit einem oder mehreren Substituenten ausgewählt aus -(C₁-C₁₂)-Alkyl und -(C₃-C₂₀)-Heteroaryl substituiert sein.

In einer Ausführungsform sind die Reste R¹, R², R³, R⁴, R^{1'}, R^{2'}, R^{3'}, R^{4'} unsubstituiert, falls sie für -(C₁-C₁₂)-Alkyl, -(C₃-C₁₂)-Cycloalkyl, oder -(C₃-C₁₂)-Heterocycloalkyl stehen, und können wie beschrieben substituiert sein, falls sie für -(C₆-C₂₀)-Aryl, oder -(C₃-C₂₀)-Heteroaryl stehen.

In einer Ausführungsform sind die Reste R¹, R², R³, R⁴, R^{1'}, R^{2'}, R^{3'}, R^{4'} unsubstituiert, falls sie für -(C₁-C₁₂)-Alkyl, -(C₃-C₁₂)-Cycloalkyl, -(C₃-C₁₂)-Heterocycloalkyl, -(C₆-C₂₀)-Aryl, oder-(C₃-C₂₀)-Heteroaryl stehen.

In einer Ausführungsform sind R¹, R², R³, R⁴, R^{1'}, R^{2'}, R^{3'}, R^{4'} jeweils unabhängig voneinander ausgewählt aus -(C₁-C₁₂)-Alkyl, -(C₃-C₁₂)-Cycloalkyl, -(C₃-C₁₂)-Heterocycloalkyl, -(C₆-C₂₀)-Aryl, -(C₃-C₂₀)-Heteroaryl;
wobei
mindestens einer der Reste R¹, R², R³, R⁴ bzw. mindestens einer der Reste R^{1'}, R^{2'}, R^{3'}, R^{4'} für einen -(C₃-C₂₀)-Heteroarylrest steht;
und
R¹, R², R³, R⁴, R^{1'}, R^{2'}, R^{3'}, R^{4'}, falls diese für -(C₁-C₁₂)-Alkyl, -(C₃-C₁₂)-Cycloalkyl, -(C₃-C₁₂)-Heterocycloalkyl, -(C₆-C₂₀)-Aryl oder -(C₃-C₂₀)-Heteroaryl stehen,
jeweils unabhängig voneinander mit einem oder mehreren der oben beschriebenen Substituenten substituiert sein können.

In einer Ausführungsform sind R¹, R², R³, R⁴, R^{1'}, R^{2'}, R^{3'}, R^{4'} jeweils unabhängig voneinander ausgewählt aus -(C₁-C₁₂)-Alkyl, -(C₆-C₂₀)-Aryl, -(C₃-C₂₀)-Heteroaryl;
wobei
mindestens einer der Reste R¹, R², R³, R⁴ bzw. mindestens einer der Reste R^{1'}, R^{2'}, R^{3'}, R^{4'} für einen -(C₃-C₂₀)-Heteroarylrest steht;
und
R¹, R², R³, R⁴, R^{1'}, R^{2'}, R^{3'}, R^{4'}, falls diese für -(C₁-C₁₂)-Alkyl, -(C₆-C₂₀)-Aryl oder -(C₃-C₂₀)-Heteroaryl stehen,
jeweils unabhängig voneinander mit einem oder mehreren der oben beschriebenen Substituenten substituiert sein können.

In einer Ausführungsform stehen mindestens zwei der Reste R¹, R², R³, R⁴ bzw. mindestens zwei der Reste R^{1'}, R^{2'}, R^{3'}, R^{4'} für einen -(C₃-C₂₀)-Heteroarylrest.

In einer Ausführungsform stehen die Reste R¹ und R³ bzw. die Reste R^{1'} und R^{3'} jeweils für einen -(C₃-C₂₀)-Heteroarylrest und können jeweils unabhängig voneinander mit einem oder mehreren der oben beschriebenen Substituenten substituiert sein. Vorzugsweise stehen die Reste R² und R⁴ bzw. die Reste R^{2'} und R^{4'} dabei nicht für einen -(C₃-C₂₀)-Heteroarylrest. Besonders bevorzugt sind die Reste R² und R⁴ bzw. die Reste R^{2'} und R^{4'} dabei ausgewählt aus -(C₁-C₁₂)-Alkyl, -(C₃-C₁₂)-Cycloalkyl, -(C₃-C₁₂)-Heterocycloalkyl, -(C₆-C₂₀)-Aryl, am meisten bevorzugt aus -(C₁-C₁₂)-Alkyl, -(C₃-C₁₂)-Cycloalkyl, -(C₆-C₂₀)-Aryl.

In einer Ausführungsform stehen die Reste R¹, R², R³ bzw. die Reste R^{1'}, R^{2'}, R^{3'} jeweils für einen -(C₃-C₂₀)-Heteroarylrest und können jeweils unabhängig voneinander mit einem oder mehreren der oben beschriebenen Substituenten substituiert sein. Vorzugsweise steht R⁴ bzw. R^{4'} dabei nicht für einen -(C₃-C₂₀)-Heteroarylrest. Besonders bevorzugt ist R⁴ bzw. R^{4'} dabei ausgewählt aus -(C₁-C₁₂)-Alkyl, -(C₃-C₁₂)-Cycloalkyl, -(C₃-C₁₂)-Heterocycloalkyl, -(C₆-C₂₀)-Aryl, am meisten bevorzugt aus -(C₁-C₁₂)-Alkyl, -(C₃-C₁₂)-Cycloalkyl, -(C₆-C₂₀)-Aryl.

In einer Ausführungsform stehen die Reste R¹, R², R³ und R⁴ bzw. R^{1'}, R^{2'}, R^{3'} und R^{4'} für einen -(C₃-C₂₀)-Heteroarylrest und können jeweils unabhängig voneinander mit einem oder mehreren der oben beschriebenen Substituenten substituiert sein.

In einer Ausführungsform sind die Reste R¹, R², R³ und R⁴ bzw. R^{1'}, R^{2'}, R^{3'} und R^{4'}, falls sie für einen Heteroarylrest stehen, jeweils unabhängig voneinander ausgewählt aus Heteroarylresten mit fünf bis zehn Ringatomen, vorzugsweise einem Heteroarylrest mit fünf oder sechs Ringatomen.

In einer Ausführungsform stellen die Reste R¹, R², R³ und R⁴ bzw. R^{1'}, R^{2'}, R^{3'} und R^{4'}, falls sie für einen Heteroarylrest stehen, einen Heteroarylrest mit sechs Ringatomen dar.

In einer Ausführungsform stellen die Reste R¹, R², R³ und R⁴ bzw. R^{1'}, R^{2'}, R^{3'} und R^{4'}, falls sie für einen Heteroarylrest stehen, einen Heteroarylrest mit fünf Ringatomen dar.

Vorzugsweise sind die Reste R¹, R², R³ und R⁴ bzw. R^{1'}, R^{2'}, R^{3'} und R^{4'}, falls sie für einen Heteroarylrest stehen, jeweils unabhängig voneinander ausgewählt aus Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl, wobei die genannten Heteroarylreste wie oben beschrieben substituiert sein können.

In einer Ausführungsform sind die Reste R¹, R², R³ und R⁴ bzw. R^{1'}, R^{2'}, R^{3'} und R^{4'}, falls sie für einen Heteroarylrest stehen, jeweils unabhängig voneinander ausgewählt aus Furyl, Thienyl, Pyrrolyl, Imidazolyl, Pyridyl, Pyrimidyl, Indolyl, wobei die genannten Heteroarylreste wie oben beschrieben substituiert sein können.

Vorzugsweise sind die Reste R¹, R², R³ und R⁴ bzw. R^{1'}, R^{2'}, R^{3'} und R^{4'}, falls sie für einen Heteroarylrest stehen, dabei jeweils unabhängig voneinander ausgewählt aus 2-Furyl, 2-Thienyl, 2-Pyrrolyl, 2-Imidazolyl, 2-Pyridyl, 2-Pyrimidyl, 2-Indolyl, wobei die genannten Heteroarylreste wie oben beschrieben substituiert sein können.

Besonders bevorzugt sind die Reste R¹, R², R³ und R⁴ bzw. R^{1'}, R^{2'}, R^{3'} und R^{4'}, falls sie für einen Heteroarylrest stehen, dabei jeweils unabhängig voneinander ausgewählt aus 2-Furyl, 2-Thienyl, N-Methyl-2-Pyrrolyl, N-Phenyl-2-Pyrrolyl, N-(2-Methoxyphenyl)-2-Pyrrolyl, 2-Pyrrolyl, N-Methyl-2-Imidazolyl, 2-Imidazolyl, 2-Pyridyl, 2-Pyrimidyl, N-Phenyl-2-Indolyl, 2-Indolyl, wobei die genannten Heteroarylreste nicht weiter substituiert sind.

In einer Ausführungsform stehen die Reste R¹ und R³ bzw. R^{1'} und R^{3'} jeweils für einen - (C₃-C₂₀)-Heteroarylrest mit fünf oder sechs Ringatomen,
wobei die Reste R² und R⁴ bzw. R^{2'} und R^{4'} jeweils unabhängig voneinander ausgewählt sind aus -(C₁-C₁₂)-Alkyl, -(C₃-C₁₂)-Cycloalkyl, -(C₃-C₁₂)-Heterocycloalkyl, -(C₆-C₂₀)-Aryl; und
R¹, R³, R^{1'}, R^{3'} sowie R², R^{2'}, R⁴, R^{4'}, falls diese für -(C₁-C₁₂)-Alkyl, -(C₃-C₁₂)-Cycloalkyl, -(C₃-C₁₂)-Heterocycloalkyl oder -(C₆-C₂₀)-Aryl stehen,
jeweils unabhängig voneinander mit einem oder mehreren der oben beschriebenen Substituenten substituiert sein können.

In einer Ausführungsform stehen die Reste R¹ und R³ bzw. R^{1'} und R^{3'} jeweils für einen-(C₃-C₂₀)-Heteroarylrest mit fünf oder sechs Ringatomen, und die Reste R² und R⁴ bzw. R^{2'} und R^{4'} stehen jeweils für -(C₁-C₁₂)-Alkyl;
wobei
R¹, R³, R^{1'}, R^{3'} sowie R², R^{2'}, R⁴, R^{4'} jeweils unabhängig voneinander mit einem oder mehreren der oben beschriebenen Substituenten substituiert sein können.

In einer Ausführungsform stehen die Reste R¹ und R³ bzw. R^{1'} und R^{3'} jeweils für einen-(C₃-C₂₀)-Heteroarylrest ausgewählt aus Furyl, Thienyl, Pyrrolyl, Imidazolyl, Pyridyl, Pyrimidyl, Indolyl,
wobei die Reste R² und R⁴ jeweils unabhängig voneinander ausgewählt sind aus -(C₁-C₁₂)-Alkyl, -(C₃-C₁₂)-Cycloalkyl, -(C₃-C₁₂)-Heterocycloalkyl, -(C₆-C₂₀)-Aryl;
und
R¹ und R³ sowie R² und R⁴, falls diese für -(C₁-C₁₂)-Alkyl, -(C₃-C₁₂)-Cycloalkyl, -(C₃-C₁₂)-Heterocycloalkyl oder -(C₆-C₂₀)-Aryl stehen,
jeweils unabhängig voneinander mit einem oder mehreren der oben beschriebenen Substituenten substituiert sein können.

In einer bevorzugten Ausführungsform ist der zweizähnige Phosphinligand eine Verbindung nach Formel **I**, wobei R¹, R², R³, R⁴ die oben genannte Bedeutung haben.

Vorzugsweise ist der zweizähnige Phosphinligand eine Verbindung nach Formel **I,**
wobei die Reste R¹ und R³ jeweils für einen -(C₃-C₂₀)-Heteroarylrest mit fünf oder sechs Ringatomen stehen;
die Reste R² und R⁴ jeweils unabhängig voneinander ausgewählt sind aus -(C₁-C₁₂)-Alkyl, -(C₃-C₁₂)-Cycloalkyl, -(C₃-C₁₂)-Heterocycloalkyl, -(C₆-C₂₀)-Aryl;
und
R¹, R², R³, R⁴ jeweils unabhängig voneinander mit einem oder mehreren der oben beschriebenen Substituenten substituiert sein können.

Besonders bevorzugt ist der zweizähnige Phosphinligand eine Verbindung nach Formel **I,**
wobei die Reste R¹ und R³ jeweils für einen -(C₃-C₂₀)-Heteroarylrest mit sechs Ringatomen stehen;
die Reste R² und R⁴ jeweils für -(C₁-C₁₂)-Alkyl stehen.

Die folgenden Verbindungen nach einer der Formeln (**8**) und (**4**) sind besonders geeignete Phosphinliganden für das erfindungsgemäße Verfahren:

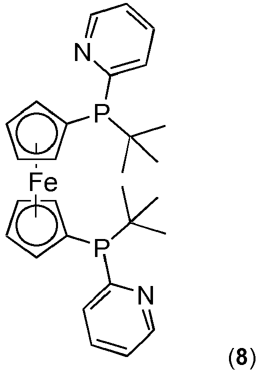

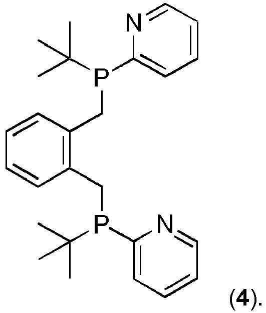

Dabei ist Verbindung **8** besonders bevorzugt.

Die in dem erfindungsgemäßen Verfahren als Edukt eingesetzten ethylenisch ungesättigten Verbindungen enthalten eine oder mehrere Kohlenstoff-Kohlenstoff-Doppelbindungen. Diese Verbindungen werden auch im Folgenden zur Vereinfachung als Olefine bezeichnet. Die Doppelbindungen können terminal oder intern sein.

Bevorzugt sind ethylenisch ungesättigte Verbindungen mit 2 bis 30 Kohlenstoffatomen, bevorzugt 2 bis 22 Kohlenstoffatomen, besonders bevorzugt 2 bis 12 Kohlenstoffatomen.

Die ethylenisch ungesättigten Verbindungen können zusätzlich zu der einen oder mehreren Doppelbindungen weitere funktionelle Gruppen enthalten. Vorzugsweise umfasst die ethylenisch ungesättigte Verbindung eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Hydroxyl-, Sulfhydryl- , Amino-, Ether-, Thioether-, Aryl-, Heteroaryl- oder Silylgruppen und/oder Halogensubstituenten. Dabei umfasst die ethylenisch ungesättigte Verbindung vorzugsweise insgesamt 2 bis 30 Kohlenstoffatome, bevorzugt 2 bis 22 Kohlenstoffatome, besonders bevorzugt 2 bis 12 Kohlenstoffatome.

In einer Ausführungsform umfasst die ethylenisch ungesättigte Verbindung keine weiteren funktionellen Gruppen außer Kohlenstoff-Kohlenstoff-Doppelbindungen.

Geeignete ethylenisch ungesättigte Verbindungen sind beispielsweise:
Ethen;
Propen;
C4-Olefine, wie 1-Buten, cis-2-Buten, trans-2-Buten, Gemisch von cis- und trans-2-Buten, Isobuten, 1,3-Butadien; Raffinat I bis III, Crack-C4
C5-Olefine, wie 1-Penten, 2-Penten, 2-Methyl-1-buten, 2-Methyl-2-buten, 2-Methyl-1,3-butadien (Isopren), 1,3-Pentadien;
C6-Olefine, wie Tetramethylethylen, 1,3-Hexadien, 1,3-Cyclohexadien;
C7-Olefine, wie 1-Methylcyclohexen, 2,4-Heptadien, Norbonadien;
C8-Olefine, wie 1-Octen, 2-Octen, Cycloocten, Di-n-buten, Di-iso-buten, 1,5-Cyclooctadien, 1,7-Octadien;
C9-Olefine, wie Tripropen;
C10-Olefine, wie Dicylcopentadien;
Undecene;
Dodecene;
interne C14-Olefine;
interne C15- bis C18-Olefine;
lineare oder verzweigte, cyclische, acyclische oder teilweise cyclische, interne C15- bis C30-Olefine;
Triisobuten, Tri-n-buten;
Terpene, wie Limonen, Geraniol, Farnesol, Pinen, Myrcen, Carvon, 3-Caren;
mehrfach ungesättigte Verbindungen mit 18 Kohlenstoffatomen, wie Linolsäure oder Linolensäure;
Ester ungesättigter Carbonsäuren, wie Vinylester von Essig- oder Propansäure, Alkylester ungesättigter Carbonsäuren, Methyl- oder Ethylester von Acrylsäure oder Methacrylsäure, Ölsäureester, Ölsäure Methyl- oder Ethylester, Ester der Linol- oder Linolensäure; Vinylverbindungen, wie Vinylacetat, Vinylcyclohexen, Styrol, alpha-Methylstyrol, 2-Isopropenylnaphthalin;
2-Methyl-2-pentenal, Methyl-3-Pentenoat, Methacrylanhydrid.

In einer Variante des Verfahrens ist die ethylenisch ungesättigte Verbindung ausgewählt aus Propen, 1-Buten, cis- und/oder trans-2-Buten, oder Mischungen davon.

In einer Variante des Verfahrens ist die ethylenisch ungesättigte Verbindung ausgewählt aus 1-Penten, cis- und/oder trans-2-Penten, 2-Methyl-1-buten, 2-Methyl-2-buten, 3-Methyl-1-buten, oder Mischungen davon.

In einer bevorzugten Ausführungsform ist die ethylenisch ungesättigte Verbindung ausgewählt aus Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, n-Octen, 1-Octen, 2-Octen, oder Mischungen davon.

In einer Variante wird ein Gemisch ethylenisch ungesättigter Verbindungen eingesetzt. Als Gemisch wird im Sinne dieser Erfindung eine Zusammensetzung bezeichnet, die wenigsten zwei verschiedene ethylenisch ungesättigte Verbindungen enthält, wobei der Anteil jeder einzelnen ethylenisch ungesättigten Verbindungen vorzugsweise wenigstens 5 Gewichts-% bezogen auf das Gesamtgewicht des Gemisches beträgt.

Vorzugsweise wird ein Gemisch ethylenisch ungesättigter Verbindungen mit jeweils 2 bis 30 Kohlenstoffatomen, bevorzugt 4 bis 22 Kohlenstoffatomen, besonders bevorzugt 6 bis 12 Kohlenstoffatomen, am meisten bevorzugt 8 bis 10 Kohlenstoffatomen eingesetzt.

Geeignete Gemische ethylenisch ungesättigter Verbindungen sind die sogenannten Raffinate I bis III. Raffinat I umfasst 40 bis 50 % iso-Buten, 20 bis 30 % 1-Buten, 10 bis 20 % cis- und trans-2-Buten, bis zu 1 % 1,3-Butadien und 10 bis 20 % n-Butan und Isobutan. Das Raffinat II ist ein Teil der beim Naphthacracken entstehenden C₄-Fraktion und besteht im Wesentlichen aus den isomeren *n*-Butenen, Isobutan und *n*-Butan nach Abtrennung von Isobuten aus Raffinat I. Raffinat III ist ein Teil der beim Naphthacracken entstehenden C₄-Fraktion und besteht im Wesentlichen aus den isomeren *n*-Butenen und *n*-Butan.

Ein weiteres geeignetes Gemisch ist Di-n-Buten, auch bezeichnet als Dibuten, DNB oder DnB. Di-n-Buten ist ein Isomerengemisch von C8-Olefinen, das aus der Dimerisierung von Gemischen aus 1-Buten, cis-2-Buten und trans-2-Buten entsteht. Technisch werden der Regel Raffinat II oder Raffinat III-Ströme einer katalytischen Oligomerisierung unterworfen, wobei die enthaltenen Butane (n/iso) unverändert hervorgehen und die enthaltenen Olefine ganz oder teilweise umgesetzt werden. Neben dem dimeren Di-n-Buten, entstehen in der Regel auch höhere Oligomere (Tributen C12, Tetrabuten C16), die nach der Reaktion destillativ abgetrennt werden. Diese können ebenfalls als Edukte eingesetzt werden.

In einer bevorzugten Variante wird ein Gemisch umfassend iso-Buten, 1-Buten, cis- und trans-2-Buten eingesetzt. Vorzugsweise umfasst das Gemisch 1-Buten, cis- und trans-2-Buten.

Die erfindungsgemäße Alkoxycarbonylierung wird durch den Pd-Komplex katalysiert. Der Pd-Komplex kann dabei in Verfahrensschritt b) entweder als präformierter Komplex umfassend Pd und den oben beschriebenen Phosphinliganden zugegeben werden, oder *in situ* aus einer Verbindung, welche Pd umfasst, und dem freien Phosphinliganden gebildet werden. Dabei wird die Verbindung, welche Pd umfasst, auch als Katalysatorvorstufe bezeichnet.

In dem Fall, dass der Katalysator *in situ* gebildet wird, kann der Ligand im Überschuss zugegeben werden, so dass im Reaktionsgemisch auch ungebundener Ligand vorliegt.

In einer Variante ist die Verbindung, welche Pd umfasst, ausgewählt aus Palladiumdichlorid (PdCl₂), Palladium(II)-acetylacetonat [Pd(acac)₂], Palladium(II)-acetat [Pd(OAc)₂], Dichloro(1,5-cyclooctadiene)palladium(II) [Pd(cod)₂Cl₂], Bis(dibenzylideneaceton)palladium [Pd(dba)₂], Bis(acetonitrile)dichloropalladium(II) [Pd(CH₃CN)₂Cl₂], Palladium(cinnamyl)dichlorid [Pd(cinnamyl)Cl₂].

Vorzugsweise handelt es sich bei der Verbindung, welche Pd umfasst, um PdCl₂, Pd(acac)₂ oder Pd(OAc)₂. Besonders geeignet ist PdCl₂.

Der Alkohol im Verfahrensschritt c) kann verzweigt oder linear sein, cyclisch, alicyclisch, teilweise zyklisch oder aliphatisch und stellt insbesondere ein C₁- bis C₃₀-Alkanol dar. Es können Monoalkohole oder Polyalkohole verwendet werden.

Der Alkohol in Verfahrensschritt c) umfasst vorzugsweise 1 bis 30 Kohlenstoffatome, bevorzugt 1 bis 22 Kohlenstoffatome, besonders bevorzugt 1 bis 12 Kohlenstoffatome. Es kann sich dabei um einen Monoalkohol oder einen Polyalkohol handeln.

Der Alkohol kann zusätzlich zu der einen oder mehreren Hydroxylgruppen weitere funktionelle Gruppen enthalten. Vorzugsweise kann der Alkohol zusätzlich eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Sulfhydryl- , Amino-, Ether-, Thioether-, Aryl-, Heteroaryl- oder Silylgruppen, und/oder Halogensubstituenten enthalten.

In einer Ausführungsform umfasst der Alkohol keine weiteren funktionellen Gruppen außer Hydroxylgruppen.

Der Alkohol kann ungesättigte und aromatische Gruppen enthalten. Vorzugsweise handelt es sich jedoch um einen aliphatischen Alkohol.

Als aliphatischer Alkohol wird im Rahmen dieser Erfindung ein Alkohol bezeichnet, der keine aromatischen Gruppen umfasst, also beispielsweise ein Alkanol, Alkenol oder Alkinol.

In einer Variante des Verfahrens ist der Alkohol im Verfahrensschritt c) ausgewählt aus der Gruppe der Monoalkohole.

In einer Variante des Verfahrens ist der Alkohol im Verfahrensschritt c) ausgewählt aus: Methanol, Ethanol, 1-Propanol, Iso-Propanol, Iso-Butanol, tert-Butanol, 1-Butanol, 2-Butanol, 1-Pentanol, 2-Pentanol, 3-Pentanol, 1-Hexanol, Cyclohexanol, Phenol, 2-Ethylhexanol, Isononanol, 2-Propylheptanol.

In einer bevorzugten Variante ist der Alkohol in Verfahrensschritt c) ausgewählt ist aus Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

In einer Variante des Verfahrens ist der Alkohol im Verfahrensschritt c) ausgewählt aus der Gruppe der Polyalkohole.

In einer Variante des Verfahrens ist der Alkohol im Verfahrensschritt c) ausgewählt aus: Diolen, Triolen, Tetraolen.

In einer Variante des Verfahrens ist der Alkohol im Verfahrensschritt c) ausgewählt aus: Cyclohexan-1,2-diol, 1,2-Ethandiol, 1,3-Propandiol, Glycerol, 1,2,4-Butantriol,2-Hydroxymethyl-1,3-Propandiol, 1,2,6-Trihydroxyhexan, Pentaerythritol, 1,1,1-Tri(hydroxymethyl)ethan, Brenzkatechin, Resorcin und Hydroxyhydrochinon.

In einer Variante des Verfahrens ist der Alkohol im Verfahrensschritt c) ausgewählt aus: Sucrose, Fructose, Mannose, Sorbose, Galactose und Glucose.

In einer bevorzugten Variante des Verfahrens ist der Alkohol im Verfahrensschritt c) ausgewählt aus Methanol, Ethanol, 1-Propanol, 1-Butanol, 1-Pentanol, 1-Hexanol.

In einer besonders bevorzugten Variante des Verfahrens ist der Alkohol im Verfahrensschritt c) ausgewählt aus: Methanol, Ethanol.

In einer besonders bevorzugten Variante des Verfahrens ist der Alkohol im Verfahrensschritt c) Methanol.

In einer Variante des Verfahrens wird der Alkohol im Verfahrensschritt c) im Überschuss eingesetzt.

In einer Variante des Verfahrens wird der Alkohol im Verfahrensschritt c) gleichzeitig als Lösungsmittel eingesetzt.

In einer Variante des Verfahrens wird ein weiteres Lösungsmittel verwendet, ausgewählt aus: Toluol, Xylol, Tetrahydrofuran (THF) oder Methylenchlorid (CH₂Cl₂).

CO wird in Schritt d) vorzugsweise bei einem CO-Partialdruck zwischen 0,1 und 10 MPa (1 bis 100 bar), bevorzugt zwischen 1 und 8 MPa (10 bis 80 bar), besonders bevorzugt zwischen 2 und 4 MPa (20 bis 40 bar) zugeführt.

Die Reaktionsmischung wird Schritt e) des erfindungsgemäßen Verfahrens vorzugsweise auf eine Temperatur zwischen 10 °C und 180 °C, bevorzugt zwischen 20 und 160 °C, besonders bevorzugt zwischen 40 und 120 °C erwärmt, um die ethylenisch ungesättigte Verbindung zu einem Ester umzusetzen.

Das molare Verhältnis von der in Schritt a) vorgelegten ethylenisch ungesättigten Verbindung zu dem in Schritt c) zugegebenen Alkohol beträgt vorzugsweise zwischen 1:1 bis 1:20, bevorzugt 1:2 bis 1:10, besonders bevorzugt 1:3 bis 1:4.

Das Massenverhältnis von Pd zu der in Schritt a) vorgelegten ethylenisch ungesättigten Verbindung beträgt vorzugsweise zwischen 0,001 und 0,5 Gew.-%, bevorzugt zwischen 0,01 und 0,1 Gew.-%, besonders bevorzugt zwischen 0,01 und 0,05 Gew.-%.

Das molare Verhältnis des zweizähnigen Phosphinliganden zu Pd beträgt vorzugsweise zwischen 0,1:1 und 400:1, bevorzugt zwischen 0,5:1 und 400:1, besonders bevorzugt zwischen 1:1 und 100:1, am meisten bevorzugt zwischen 2:1 und 50:1.

### Beschreibung der Abbildungen

**Figur 1****:** Einfluss der Palladiumvorstufe auf die Methoxycarbonylierung von Ethylen mit den Liganden **3, 4** und **8.**
**Figur 2****:** Säurefreie Methoxycarbonylierung von Ethen mit den Liganden **3, 4** und **8**

### Beispiele

Im Folgenden wird die Erfindung anhand von Ausführungsbeispielen näher beschrieben.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, und Pierre Granger, Pure Appl. Chem., 2001, 73, 1795-1818; Robin K. Harris, Edwin D. Becker, Sonia M. Cabral de Menezes, Pierre Granger, Roy E. Hoffman und Kurt W. Zilm, Pure Appl. Chem., 2008, 80, 59-84).

Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Herstellung von Vorstufe E

### Darstellung von Chlor-2-pyridyl-tert-butylphosphin

Der Grignard für die Synthese von Chlor-2-pyridyl-t-butylphosphin wird nach der "Knochelmethode" mit Isopropylmagnesiumchlorid dargestellt (Angew. Chem. 2004, 43, 2222-2226). Die Aufarbeitung erfolgt nach der Methode von Budzelaar (Organometallics 1990, 9, 1222-1227).

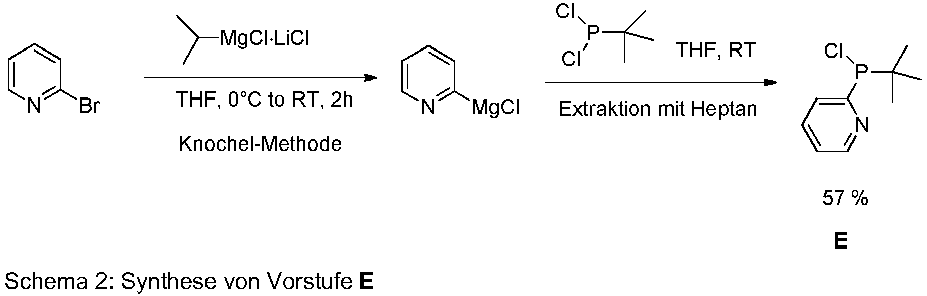

In einen 50 ml Rundkolben mit Magnetrührer und Septum werden unter Argon 8.07 ml einer 1.3 M Isopropylmagnesium-chloridlösung (Knochel-Reagenz) gegeben und auf -15 °C gekühlt. Danach werden zügig 953.5 µl (10 mmol) 2-Bromopyridin zu getropft. Die Lösung wird sofort gelb. Man lässt auf -10 °C aufwärmen. Der Umsatz der Reaktion wird wie folgt bestimmt: man entnimmt ca. 100 µl Lösung und gibt sie in 1 ml einer gesättigten Ammoniumchloridlösung. Wenn die Lösung "sprudelt", dann hat sich noch nicht viel Grignard gebildet. Die wässrige Lösung wird mit einer Pipette Ether extrahiert und die organische Phase über Na₂SO₄ getrocknet. Von der etherischen Lösung wird ein GC aufgenommen. Wenn sich viel Pyridin gebildet hat im Vergleich zu 2-Bromopyridin, dann hat man hohe Umsätze. Bei -10 °C hat sich wenig umgesetzt. Nach Aufwärmen auf Raumtemperatur und 1-2 stündigen Rühren wird die Reaktionslösung braungelb. Ein GC-Test zeigt vollständigen Umsatz. Jetzt kann die Grignardlösung zu einer Lösung von 1.748 g (11 mmol) Dichloro-tert-butylphosphin in 10 ml THF, die vorher auf-15 °C gekühlt worden ist, mit einer Spritzenpumpe langsam zu getropft werden. Wichtig ist, dass die Dichloro-tert-butylphosphinlösung gekühlt wird. Bei Raumtemperatur würde man erhebliche Mengen an Dipyridyl-tert-butylphosphin erhalten. Es entsteht anfangs eine klare gelbe Lösung, die dann trübe wird. Man lässt auf Raumtemperatur aufwärmen und über Nacht rühren. Man entfernt das Lösungsmittel im Hochvakuum und erhält einen weißlichen Feststoff, der braune Stellen enthält. Der Feststoff wird mit 20 ml Heptan suspendiert und der Feststoff wird im Ultraschallbad zerkleinert. Nach dem Absetzen lassen des weißen Feststoffes wird die Lösung dekantiert. Der Vorgang wird zwei Mal mit je 10-20 ml Heptan wiederholt. Nach dem Einengen der Heptanlösung im Hochvakuum wird diese unter Vakuum destilliert. Bei 4.6 mbar, 120 °C Ölbad- und 98 °C Übergangstemperatur kann das Produkt destilliert werden. Man erhält 1.08 g eines farblosen Öls. (50%).
Analytische Daten: ¹H NMR (300 MHz, C₆D₆): δ 8.36 (m, 1H, Py), 7.67 (m, 1H, Py), 7.03-6.93 (m, 1H, Py), 6.55-6.46 (m, 1H, Py), 1.07 (d, J = 13.3 Hz, 9H, t-Bu)
¹³C NMR (75 MHz, C₆D₆): δ 162.9, 162.6, 148.8, 135.5, 125.8, 125.7, 122.8, 35.3, 34.8, 25.9 und 25.8.
³¹P NMR (121 MHz, C₆D₆) δ 97.9.
MS (EI) *m*:*z* (relative Intensität) 201 (M⁺,2), 147(32), 145 (100), 109 (17), 78 (8), 57.1 (17).

### Herstellung von Verbindung 8

### Darstellung von 1,1'-Bis(tert-butyl-2-pyridylphosphino)ferrocen

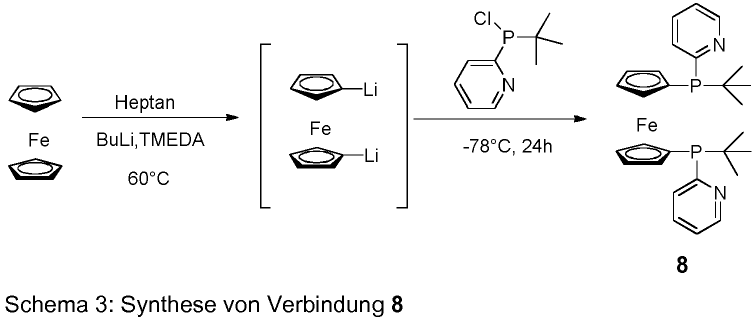

### Variante A:

474.4 mg (2.55 mmol) sublimiertes Ferrocen wird in einen 50 ml Rundkolben mit Magnetrührer und Septum eingewogen und sekuriert. Nach Zugabe von 15 ml Heptan hat sich das Ferrocen vollständig aufgelöst. Dann werden 841 µl Tetramethylethylendiamin (1.1 eq, 5.61 mmol) auf einmal zugegeben und 2.04 ml BuLi (2.5 M in Hexan, 2.0 eq.5.1 mmol) zu getropft. Nach 2-3 Stunden fällt ein oranger Niederschlag aus. Man lässt über Nacht Rühren, dekantiert die Heptanlösung und wäscht den orangen Feststoff zwei Mal mit Heptan. Dann werden erneut 10 ml Heptan zugegeben und die Suspension wird auf -70 °C gekühlt. 1.08 g (2.1 äq, 5.36 mmol) Chlor-2-pyridyl-tert-butylphosphin wird in 7 ml Heptan gelöst. Die Lösung ist trüb und muss über Celite filtriert werden. Etwas unlöslicher weißer Feststoff hat sich gebildet. Diese Lösung wird zur Dilithiumferrocenlösung zu getropft. Während des Aufwärmens auf Raumtemperatur hellt sich die orange Suspension auf. Zur Vervollständigung der Reaktion wird die Reaktionslösung unter Rückfluss erwärmt für ca. 1 Stunde. Es hat sich eine klare orange Lösung und weißer Niederschlag gebildet.

Zur Suspension werden 7 ml mit Argon gesättigtes Wasser gegeben. Der weiße Niederschlag löst sich auf. Nach Entfernen der wässrigen Phase wird der Vorgang zwei Mal wiederholt. Dabei wird die Heptanphase trüb. Nach vollständigem Entfernen der organischen Phase im Hochvakuum, bleibt ein oranger öliger Rückstand zurück. Dieser wird in 10 ml Ether aufgenommen und über Na₂SO₄ getrocknet. (Rohausbeute 913 mg) Bei -28 °C bilden sich über Nacht weder ein Niederschlag noch Kristalle. Auch eine Mischung aus Diethylether und Heptan führt bei -28 °C nicht zur Kristallisation. Ein ³¹P NMR der Lösung zeigt wieder den Produktpeak jetzt bei 7,39 ppm und ein Signal bei 40,4 ppm. Das Produkt kann durch Säulenchromatographie gereinigt werden. Die Etherlösung wird auf eine mit Diethylether eluierte kurze Säule unter Argon aufgetragen. Die orange Produktfront läuft ganz vorne weg und kann leicht aufgefangen werden. Nach Entfernen des Ethers erhält man 241 mg (16 %) eines orangen zähen Öls in ca. 95%iger Reinheit.

### Variante B:

Ansatzgröße: 650.17 mg (3.495 mol) Ferrocen (sublimiert), 2.8 ml (2 eq, 6.99 mmol) 2.5 M BuLi (n-Butyllithium), 1.1 ml (2.1 eq, 7.3 mmol) Tetramethylethylendiamin und 1.48 g (2.1 eq, 7.34 mmol) Chlor-2-pyridyl-tert-butylphosphin.

Das Dilithiumsalz des Ferrocens wird wieder in 15 ml Heptan hergestellt. Das Chlor-2-pyridyl-tert.-butylphosphin wird anstelle von Heptan in 10 ml THF gelöst, weil sich das Chlorphosphin besser in THF löst. Die Aufarbeitung wurde ebenfalls optimiert: Nach dem Kochen unter Rückfluss wird die Reaktionsmischung mit nur 1 ml H₂O gequencht und das Lösemittel (Heptan und THF) vollständig im Hochvakuum entfernt. Der dunkelgelborange zähe Feststoff wird in 8 ml H₂O und 15 ml Diethylether aufgenommen und 1 Minute gerührt. Nach Phasentrennung wird die wässrige Phase via Spritze entfernt und die organische Phase drei Mal mit H₂O gewaschen. Die organische Phase wird über Na₂SO₄ getrocknet und filtriert. 3 Mal mit je 10 ml Diethylether wird das Produkt aus den Na₂SO₄ herausgewaschen, bis die Lösung fast farblos ist. Die dunkelorange Lösung wird auf 10 ml Volumen eingeengt und über eine Säule mit Kieselgel 60 unter Argon geschickt. Als Laufmittel wird wieder Diethylether verwendet. Das Filtrat ist wesentlich heller und orange. Nach Entfernung des Lösungsmittels erhält man 1.16 g eines zähen orangen Feststoffes. (64%)

### Darstellung von Verbindung 4 (α,α'-Bis(2-pyridyl(t-butyl)phosphino)o-xylen)

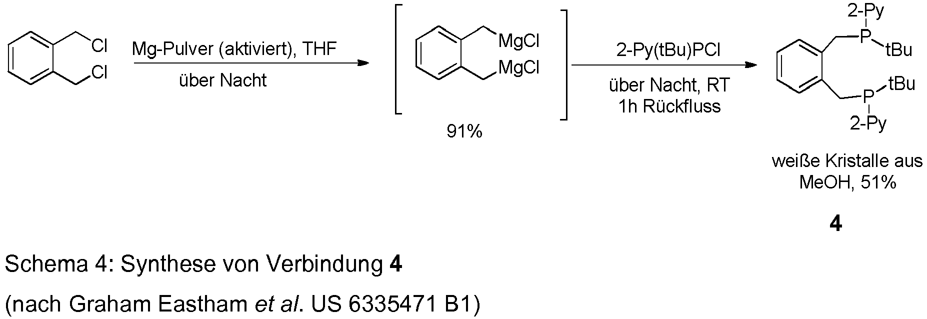

675 mg (27,8 mmol, 4 eq) Mg-Pulver werden in der Glovebox in einem 250 ml-Rundkolben mit Stickstoffhahn und Magnetrührkern eingewogen und mit einem Septum verschlossen. Man legt an den Rundkolben Hochvakuum (ca. 5x10⁻² mbar) an und erwärmt für 45 Minuten auf 90 °C. Nach Abkühlen auf Raumtemperatur werden 2 Körnchen lod zugegeben und in 20 ml THF gelöst. Man rührt die Suspension ca. 10 Minuten bis die gelbe Farbe des Iods verschwunden ist. Nach Absetzen des Magnesiumpulvers wird die trübe THF-Lösung dekantiert und das aktivierte Magnesiumpulver 2 Mal mit 1-2 ml THF gewaschen. Dann werden erneut 20 mL frisches THF zugegeben. Bei Raumtemperatur wird eine Lösung von 1,21 g (6,9 mmol) α,α'-Dichloro-o-xylene in 70 ml THF mit der Spritzenpumpe langsam zugetropft. Die THF-Lösung verfärbt sich langsam dunkel. Am nächsten Tag wird die THF-Suspension von dem nicht umgesetzten Magnesiumpulver filtriert und der Gehalt an Grignardverbindung wird wie folgt bestimmt:
1 ml Grignardlösung wird in einer gesättigten wässrigen Lösung von NH₄Cl gequencht und mit Ether extrahiert. und mit Na₂SO₄ getrocknet

### Quantitative Bestimmung des Gehalts der Grignardlösung:

1 ml Grignardlösung wird mit 2 ml 0.1M HCl gequenched und die überschüssige Säure mit 0.1M NaOH titriert. Als Indikator ist eine wässrige 0.04%ige Bromkresollösung geeignet. Farbumschlag geht von gelb nach blau. Es sind 0.74 ml an 0.1 M NaOH verbraucht worden. 2 ml-0.74 ml = 1.26 ml, das entspricht 0.126 mmol Grignardverbindung. Da ein Digrignard vorliegt, ist die Grignardlösung 0.063M. Das sind über 90% Ausbeute.

In einem 250mL Dreihalskolben mit Rückflusskühler und Magnetrührer werden unter Argon 1.8 g (8.66 mmol) Chlorphosphin (2-Py(tBu)PCI) in 10 mL THF gelöst und auf -60 °C gekühlt. Dann werden 55 mL der oben bestimmten Grignardlösung (0.063M, 3.46mmol) langsam bei dieser Temperatur mit der Spritzenpumpe zugetropft. Die Lösung bleibt zunächst klar und wird dann intensiv gelb. Nach 1.5 Stunden wird die Lösung trüb. Man lässt über Nacht auf Raumtemperatur aufwärmen und man erhält eine klare gelbe Lösung. Zur Vervollständigung der Reaktion erhitzt man 1 Stunde unter Rückfluss. Nach Abkühlen gibt man 1 mL H₂O dazu und die Lösung entfärbt sich und wird milchig trüb. Nach Entfernen von THF im Hochvakuum erhält man einen zähen, hellgelben Feststoff. Man gibt 10 mL Wasser und 10 mL Ether dazu und erhält zwei homogene klare Phasen, die sich gut trennen lassen. Die wäßrige Phase wird 2 Mal mit Ether extrahiert. Nach Trocknen der organischen Phase mit Na₂SO₄ wird der Ether im Hochvakuum entfernt und man erhält einen zähen fast farblosen Feststoff. Dieser wird in 5 mL MeOH unter Erwärmen auf dem Wasserbad gelöst und über Celite filtriert. Bei -28 °C erhält man über Nacht 772 mg Produkt in Form von weißen Kristallen. (51%). Aus der Mutterlösung konnten nach Einengen nochmals 100 mg isoliert werden. Die Gesamtausbeute beträgt 57,6 %.
¹H NMR (300 MHz, C₆D₆): δ 8.58 (m, 2H, Py), 7.31-7.30 (m, 2H, Benzol), 7.30-7.22 (m, 2H, Py), 6.85-6.77 (m, 2H, Py), 6.73 (m, 2H, Benzol), 6.57-6.50 (m, 2H, py), 4.33(dd, J = 13.3 und 4.3 Hz, 2H, CH₂), 3.72-3.62 (m, 2H, CH₂), 121(d, J = 11.8 Hz, 18H, tBu).
¹³C NMR (75 MHz, C₆D₆): δ 161.3, 161.1, 149.6, 137.8, 137.7, 134.5, 133.3, 132.7, 131.4, 131.3, 125.7, 122.9, 30.7, 30.5, 28.2, 28.0, 26.5, 26.4, 26.2, und 26.1.
³¹P NMR (121 MHz, C₆D₆) δ 8.8.
EA berechnet für C₂₆H₃₄N₂P₂: C, 71.54; H, 7.85; N, 6.56; P,14.35. Gefunden: C, 71.21; H, 7.55; N, 6.56; P, 14.35.

### Hochdruckexperimente

### Einsatzstoffe:

Methanol (MeOH)
Ethen (auch als Ethylen bezeichnet)

### Allgemeine Vorschrift zur Durchführung der Hochdruckexperimente:

### Allgemeine Versuchsbeschreibung für Reaktionen im Batchversuch:

Abhängig von der Palladiumvorstufe werden 0,04 mol% bezogen auf das Ethylen unter Argon und 0,16 mol% des entsprechenden Liganden in einen gasdicht verschließbaren 25 ml Parr-Reaktor (Parr-Autoklav) eingewogen. Es werden 5 ml Methanol zugesetzt. Dann werden 1 g Ethylen (35,7 mmol) in den Autoklaven überführt (Kontrolle über Wiegen des Autoklaven). Der Autoklav wird auf 80 °C erhitzt. Der Eigendruck des Ethylens bei 80 °C beträgt nun 20 bar. Es werden nun 30 bar CO aufgepresst. Bei dieser Temperatur wird der Autoklav 20 h gerührt und der Druckabfall mittels eines Drucksensors und der Software Specview von Parr Instruments gemessen. Die im Diagramm angegebenen Ausbeuten an Produkt stimmen mit dem Gasverbrauch überein. Anschließend wird der Autoklav auf Raumtemperatur heruntergekühlt und der Druck abgelassen. Der Inhalt des Autoklaven wird in ein 50 ml Schlenkgefäß überführt und es werden 1 ml Isooctan als interner Standard zugesetzt. Die Ausbeute an Methylpropionat wird mittels GC Analyse bestimmt.

### Analytik:

GC Analytik der Produkte aus Ethen: Für die GC Analyse wird ein Chromatograph der Firma Agilent GC Agilent 7890A mit einer 30 m HP Säule verwendet. Temperaturprofil: 35 °C, 10 min; 10 °C/min zu 200 °C, 16.5 min; das Injektionsvolumen beträgt 1 µl mit einem Split von 50:1. Retentionszeit von Methylpropionat : 6.158 min

### Analytik Methanol

Methanol wurde in einer Lösungsmitteltrocknungsanlage vorbehandelt: Pure Solv MD-/ Lösungsmittel Purification System, Firma Innovative Technology Inc. One Industrial Way, Amesbury MA 01013

### Wasserwerte:

Bestimmt mit Karl Fischer Tritration: TitraLab 580-TIM580, Firma Radiometer Analytical SAS (Karl- Fischer Titration), Wassergehalt: Messbereiche, 0,1-100% w/w, gemessener Wassergehalt: 0,13889%

### Eingesetzt wurden:

Technisches Methanol Applichem: Nr. A2954,5000, Chargennummer: LOT: 3L005446 Wassergehalt max. 1 %
Methanol Acros Organics (über Molsieb): Wassergehalt 0,005 %, Codenummer: 364390010, Chargennummer: LOT 1370321

TON: Turnovernumber, definiert als mol Produkt pro mol Katalysatormetall
TOF: Turnoverfrequenz, definiert als TON pro Zeit für das Erreichen eines bestimmten Umsatzes, z.B. 50 %
Das n/iso-Verhältnis gibt das Verhältnis von endständig zu Estern umgesetzten Olefinen zu innenständig zu Estern umgesetzten Olefinen an.

Die im Folgenden angegebenen n-Selektivitäten beziehen sich auf den Anteil der endständigen Methoxycarbonylierung bezogen auf die Gesamtausbeute an Methoxycarbonylierungsprodukten.

### Beispiel Ethylen

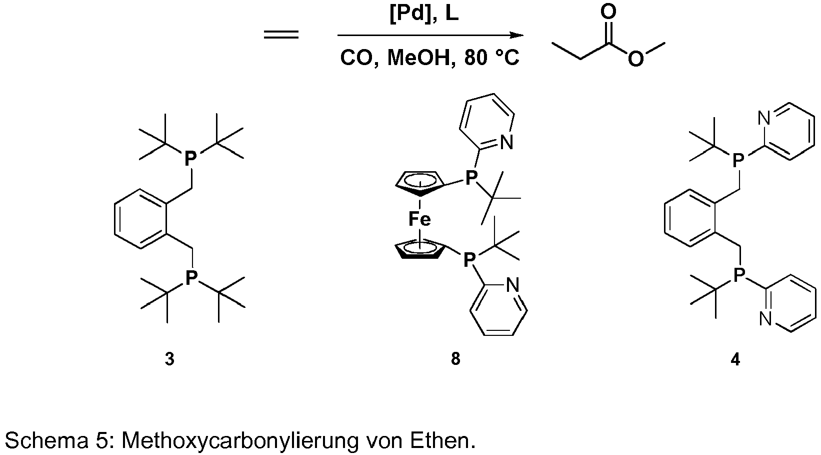

**PdCl₂/3 (Vergleichsbeispiel):** Ein 25 ml Parr-Autoklav wird unter Argon mit PdCl₂ (2,53 mg, 0,04 mol% bezogen auf die Stoffmenge von Ethylen) und 3 (22,5 mg, 0,16 mol% bezogen auf die Stoffmenge von Ethylen) und 5 ml Methanol befüllt. Dann werden 1 g (35,7 mmol) Ethylen in den Autoklaven überführt. Die Massekontrolle erfolgt über das Wiegen des Autoklaven. Der Autoklav wird auf 80 °C erhitzt. Der Druck im Autoklaven beträgt dann bei 80 °C 20 bar. Jetzt werden 30 bar CO aufgepreßt. Es wird 20 h bei 80 °C gerührt und der Druckabfall im Autoklaven gemessen. Dann wird der Autoklav abgekühlt und der Restdruck abgelassen Der Inhalt des Autoklaven wird nun in ein 50 ml Schlenkgefäß überführt und mit 1 ml Isooctan als internen Standard versetzt. Es erfolgt eine GC Analyse zur Ausbeutebestimmung. Die Ausbeute beträgt 20 %.

**PdCl₂/8:** Ein 25 ml Parr-Autoklav wird unter Argon mit PdCl₂ (2,53 mg, 0,04 mol% bezogen auf die Stoffmenge von Ethylen) und **8** (29,5 mg, 0,16 mol% bezogen auf die Stoffmenge von Ethylen) und 5 ml Methanol befüllt. Dann werden 1 g (35,7 mmol) Ethylen in den Autoklaven überführt. Die Massekontrolle erfolgt über das Wiegen des Autoklaven. Der Autoklav wird auf 80 °C erhitzt. Der Druck im Autoklaven beträgt dann bei 80 °C 20 bar. Jetzt werden 30 bar CO aufgepreßt. Es wird 20 h bei 80 °C gerührt und der Druckabfall im Autoklaven gemessen. Dann wird der Autoklav abgekühlt und der Restdruck abgelassen Der Inhalt des Autoklaven wird nun in ein 50 ml Schlenkgefäß überführt und mit 1 ml Isooctan als internen Standard versetzt. Es erfolgt eine GC Analyse zur Ausbeutebestimmung. Die Ausbeute beträgt 100 %.

**PdCl₂/4:** Ein 25 ml Parr-Autoklav wird unter Argon mit PdCl₂ (2,53 mg, 0,04 mol% hier und im Folgenden immer bezogen auf die Stoffmenge von Ethylen) und **4** (24,9 mg, 0,16 mol% hier und im Folgenden immer bezogen auf die Stoffmenge von Ethylen) und 5 ml Methanol befüllt. Dann werden 1 g (35,7 mmol) Ethylen in den Autoklaven überführt. Die Massekontrolle erfolgt über das Wiegen des Autoklaven. Der Autoklav wird auf 80 °C erhitzt. Der Druck im Autoklaven beträgt dann bei 80 °C 20 bar. Jetzt werden 30 bar CO aufgepreßt. Es wird 20 h bei 80 °C gerührt und der Druckabfall im Autoklaven gemessen. Dann wird der Autoklav abgekühlt und der Restdruck abgelassen Der Inhalt des Autoklaven wird nun in ein 50 ml Schlenkgefäß überführt und mit 1 ml Isooctan als internen Standard versetzt. Es erfolgt eine GC Analyse zur Ausbeutebestimmung. Die Ausbeute beträgt 100 %.

**Pd(acac)₂/3 (Vergleichsbeispiel):** Ein 25 ml Parr-Autoklav wird unter Argon mit Pd(acac)₂ (4,34 mg, 0,04 mol%) und 3 (22,5 mg, 0,16 mol%) und 5 ml Methanol befüllt. Dann werden 1 g (35,7 mmol) Ethylen in den Autoklaven überführt. Die Massekontrolle erfolgt über das Wiegen des Autoklaven. Der Autoklav wird auf 80 °C erhitzt. Der Druck im Autoklaven beträgt dann bei 80 °C 20 bar. Jetzt werden 30 bar CO aufgepreßt. Es wird 20 h bei 80 °C gerührt und der Druckabfall im Autoklaven gemessen. Dann wird der Autoklav abgekühlt und der Restdruck abgelassen Der Inhalt des Autoklaven wird nun in ein 50 ml Schlenkgefäß überführt und mit 1 ml Isooctan als internen Standard versetzt. Es erfolgt eine GC Analyse zur Ausbeutebestimmung. Ausbeute an Produkt ist nicht nachweisbar.

**Pd(acac)₂/8:** Ein 25 ml Parr-Autoklav wird unter Argon mit Pd(acac)₂ (4,34 mg, 0,04 mol%) und **8** (29,5 mg, 0,16 mol%) und 5 ml Methanol befüllt. Dann werden 1 g (35,7 mmol) Ethylen in den Autoklaven überführt. Die Massekontrolle erfolgt über das Wiegen des Autoklaven. Der Autoklav wird auf 80 °C erhitzt. Der Druck im Autoklaven beträgt dann bei 80 °C 20 bar. Jetzt werden 30 bar CO aufgepreßt. Es wird 20 h bei 80 °C gerührt und der Druckabfall im Autoklaven gemessen. Dann wird der Autoklav abgekühlt und der Restdruck abgelassen Der Inhalt des Autoklaven wird nun in ein 50 ml Schlenkgefäß überführt und mit 1 ml Isooctan als internen Standard versetzt. Es erfolgt eine GC Analyse zur Ausbeutebestimmung. Die Ausbeute beträgt 60 %

**Pd(acac)₂/4:** Ein 25 ml Parr-Autoklav wird unter Argon mit Pd(acac)₂ (4,34 mg, 0,04 mol%) und **4** (24,9 mg, 0,16 mol%) und 5 ml Methanol befüllt. Dann werden 1 g (35,7 mmol) Ethylen in den Autoklaven überführt. Die Massekontrolle erfolgt über das Wiegen des Autoklaven. Der Autoklav wird auf 80 °C erhitzt. Der Druck im Autoklaven beträgt dann bei 80 °C 20 bar. Jetzt werden 30 bar CO aufgepreßt. Es wird 20 h bei 80 °C gerührt und der Druckabfall im Autoklaven gemessen. Dann wird der Autoklav abgekühlt und der Restdruck abgelassen Der Inhalt des Autoklaven wird nun in ein 50 ml Schlenkgefäß überführt und mit 1 ml Isooctan als internen Standard versetzt. Es erfolgt eine GC Analyse zur Ausbeutebestimmung. Die Ausbeute beträgt 29 %.

**Pd(OAc)₂/8:** Ein 25 ml Parr-Autoklav wird unter Argon mit Pd(OAc)₂ (3,2 mg, 0,04 mol%) und **8** (29,5 mg, 0,16 mol%) und 5 ml Methanol befüllt. Dann werden 1 g (35,7 mmol) Ethylen in den Autoklaven überführt. Die Massekontrolle erfolgt über das Wiegen des Autoklaven. Der Autoklav wird auf 80 °C erhitzt. Der Druck im Autoklaven beträgt dann bei 80 °C 20 bar. Jetzt werden 30 bar CO aufgepreßt. Es wird 20 h bei 80 °C gerührt und der Druckabfall im Autoklaven gemessen. Dann wird der Autoklav abgekühlt und der Restdruck abgelassen Der Inhalt des Autoklaven wird nun in ein 50 ml Schlenkgefäß überführt und mit 1 ml Isooctan als internen Standard versetzt. Es erfolgt eine GC Analyse zur Ausbeutebestimmung. Die Ausbeute beträgt 58 %.

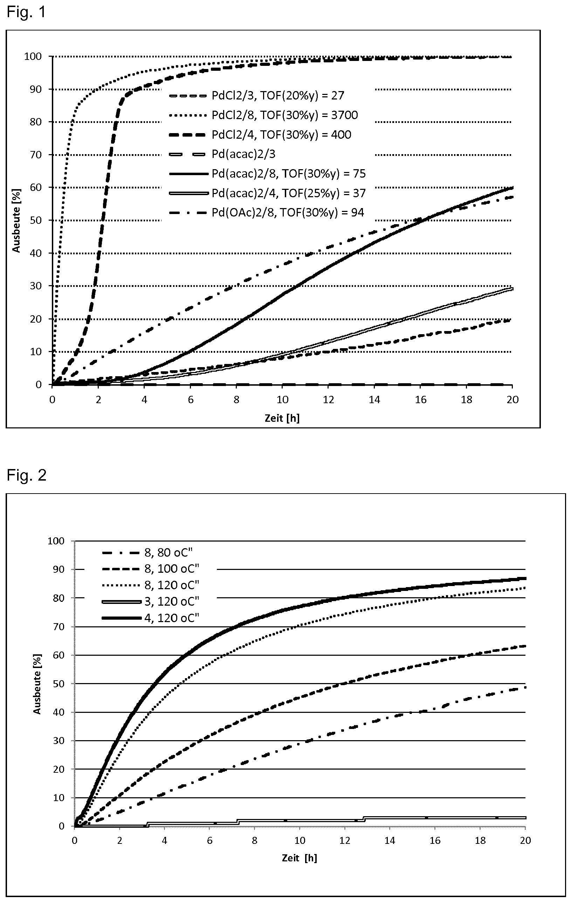

### Die Ergebnisse sind in Figur 1 dargestellt

### Figur 1: Einfluss der Palladiumvorstufe auf die Methoxycarbonylierung von Ethylen mit den Liganden 3, 4 und 8.

Wie deutlich zu erkennen ist, wird mit der Kombination PdCl₂ / Ligand **8** ohne Zusatz einer Säure bereits nach ca. 2 Stunden eine Ausbeute von > 90 % an Methylpropionat erreicht mit einer Turnover-Frequenz von 3700 mol Produkt/(mol Pd*h) bezogen auf eine Ausbeute von 30 %. Ähnlich gute Werte werden mit dem Liganden **4** erreicht. Hier werden 90 % Ausbeute nach ca. 3 Stunden mit einer Turnover-Frequenz von 400. Der Vergleichsligand DTBPMB **(3)** zeigt im Vergleich dazu nach 20 Stunden erst eine Ausbeute von ca. 20 % bei einer Turnover-Frequenz von 27. Auch die Verwendung von Pd-Acetylacetonat oder Pd-Acetat als Metallprecursor in Kombination mit den erfindungsgemäß einzusetzenden Liganden führt im säurefreien System noch zu messbaren Ausbeuten, während Ligand **3** nicht mehr katalytisch aktiv ist.

### Figur 2: Säurefreie Methoxycarbonylierung von Ethen mit den Liganden 3, 4 und 8

In **Figur 2** sind Ergebnisse zur säurefreien Methoxycarbonylierung von Ethylen mit den Liganden **3, 4** und **8** dargestellt. Bezugspunkt ist die Methoxycarbonylierung mit Ligand **8** und der Pd-Verbindung Palladiumacetat Pd(OAc)₂ bei 80 °C. Durch Temperaturerhöhung auf 120 °C kann die Ausbeute an Methylpropionat in 20 Stunden von 50 % auf 84 % gesteigert werden. Mit dem Liganden **4** können bei 120 °C nach 20 Stunden sogar 87 % Ausbeute erreicht werden. Der Vergleichsligand DTBPMB **(3)** zeigt nur eine Ausbeute von 3 % im säurefreien System. Die Versuche sind im Folgenden eingehend beschrieben.

**Pd(OAc)₂/8:** Ein 25 ml Parr-Autoklav wird unter Argon mit Pd(OAc)₂ (3,2 mg, 0,04 mol%) und **8** (29,5 mg, 0,16 mol%) und 5 ml Methanol befüllt. Dann werden 1 g (35,7 mmol) Ethylen in den Autoklaven überführt. Die Massekontrolle erfolgt über das Wiegen des Autoklaven. Jetzt werden 30 bar CO aufgepresst Der Autoklav wird auf 80 °C erhitzt. Es wird 20 h bei 80 °C gerührt und der Druckabfall im Autoklaven gemessen. Dann wird der Autoklav abgekühlt und der Restdruck abgelassen Der Inhalt des Autoklaven wird nun in ein 50 ml Schlenkgefäß überführt und mit 1 ml Isooctan als internen Standard versetzt. Es erfolgt eine GC Analyse zur Ausbeutebestimmung. Die Ausbeute beträgt 50 %.

**Pd(OAc)₂/8:** Ein 25 ml Parr-Autoklav wird unter Argon mit Pd(OAc)₂ (3,2 mg, 0,04 mol%) und **8** (29,5 mg, 0,16 mol%) und 5 ml Methanol befüllt. Dann werden 1 g (35,7 mmol) Ethylen in den Autoklaven überführt. Die Massekontrolle erfolgt über das Wiegen des Autoklaven. Jetzt werden 30 bar CO aufgepreßt Der Autoklav wird auf 100 °C erhitzt. Es wird 20 h bei 100 °C gerührt und der Druckabfall im Autoklaven gemessen. Dann wird der Autoklav abgekühlt und der Restdruck abgelassen Der Inhalt des Autoklaven wird nun in ein 50 ml Schlenkgefäß überführt und mit 1 ml Isooctan als internen Standard versetzt. Es erfolgt eine GC Analyse zur Ausbeutebestimmung. Die Ausbeute beträgt 64 %.

**Pd(OAc)₂/8:** Ein 25 ml Parr-Autoklav wird unter Argon mit Pd(OAc)₂ (3,2 mg, 0,04 mol%) und **8** (29,5 mg, 0,16 mol%) und 5 ml Methanol befüllt. Dann werden 1 g (35,7 mmol) Ethylen in den Autoklaven überführt. Die Massekontrolle erfolgt über das Wiegen des Autoklaven. Jetzt werden 30 bar CO aufgepreßt Der Autoklav wird auf 120 °C erhitzt. Es wird 20 h bei 120 °C gerührt und der Druckabfall im Autoklaven gemessen. Dann wird der Autoklav abgekühlt und der Restdruck abgelassen Der Inhalt des Autoklaven wird nun in ein 50 ml Schlenkgefäß überführt und mit 1 ml Isooctan als internen Standard versetzt. Es erfolgt eine GC Analyse zur Ausbeutebestimmung. Die Ausbeute beträgt 84 %.

**Pd(OAc)₂/3 (Vergleichsbeispiel):** Ein 25 ml Parr-Autoklav wird unter Argon mit Pd(OAc)₂ (3,2 mg, 0,04 mol%) und **3** (22,5 mg, 0,16 mol%) und 5 ml Methanol befüllt. Dann werden 1 g (35,7 mmol) Ethylen in den Autoklaven überführt. Die Massekontrolle erfolgt über das Wiegen des Autoklaven. Jetzt werden 30 bar CO aufgepreßt. Der Autoklav wird auf 120 °C erhitzt. Es wird 20 h bei 120 °C gerührt und der Druckabfall im Autoklaven gemessen. Dann wird der Autoklav abgekühlt und der Restdruck abgelassen Der Inhalt des Autoklaven wird nun in ein 50 ml Schlenkgefäß überführt und mit 1 ml Isooctan als internen Standard versetzt. Es erfolgt eine GC Analyse zur Ausbeutebestimmung. Die Ausbeute beträgt 3 %.

**Pd(OAc)₂/4:** Ein 25 ml Parr-Autoklav wird unter Argon mit Pd(OAc)₂ (3,2 mg, 0,04 mol%) und **4** (24,9 mg, 0,16 mol%) und 5 ml Methanol befüllt. Dann werden 1 g (35,7 mmol) Ethylen in den Autoklaven überführt. Die Massekontrolle erfolgt über das Wiegen des Autoklaven. Jetzt werden 30 bar CO aufgepreßt Der Autoklav wird auf 120 °C erhitzt. Es wird 20 h bei 120 °C gerührt und der Druckabfall im Autoklaven gemessen. Dann wird der Autoklav abgekühlt und der Restdruck abgelassen Der Inhalt des Autoklaven wird nun in ein 50 ml Schlenkgefäß überführt und mit 1 ml Isooctan als internen Standard versetzt. Es erfolgt eine GC Analyse zur Ausbeutebestimmung. Die Ausbeute beträgt 87 %.

## Patentansprüche

1. Verfahren umfassend die Verfahrensschritte:
a) Vorlegen einer ethylenisch ungesättigten Verbindung;
b) Zugabe eines Ligand-Metall-Komplexes umfassend Pd und einen zweizähnigen Phosphinliganden,
oder Zugabe eines zweizähnigen Phosphinliganden und einer Verbindung, welche Pd umfasst;
c) Zugabe eines Alkohols;
d) Zuführen von CO;
e) Erwärmen des Reaktionsgemisches, wobei die ethylenisch ungesättigte Verbindung zu einem Ester umgesetzt wird,
wobei dem Reaktionsgemisch weniger als 0,1 mol-%, bezogen auf die Stoffmenge der ethylenisch ungesättigten Verbindung, Brønstedsäuren mit einer Säurestärke von pKs ≤ 3 zugesetzt werden,
**dadurch gekennzeichnet, dass** der Phosphinligand an wenigstens einem Phosphoratom mit wenigstens einem Heteroarylrest substituiert ist;
wobei der zweizähnige Phosphinligand ausgewählt ist aus einer Verbindung nach einer der Formeln (**I**) und (**II**) 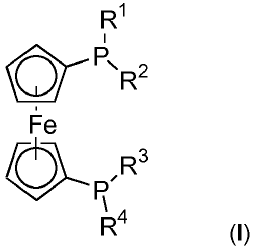 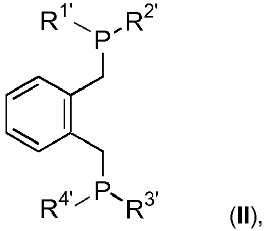 wobei
R¹, R², R³, R⁴, R^{1'}, R^{2'}, R^{3'}, R^{4'} jeweils unabhängig voneinander ausgewählt sind aus -(C₁-C₁₂)-Alkyl, -(C₃-C₁₂)-Cycloalkyl, -(C₃-C₁₂)-Heterocycloalkyl, -(C₆-C₂₀)-Aryl, -(C₃-C₂₀)-Heteroaryl;
mindestens einer der Reste R¹, R², R³, R⁴ bzw. mindestens einer der Reste R^{1'}, R^{2'}, R^{3'}, R^{4'} für einen -(C₃-C₂₀)-Heteroarylrest steht;
und
R¹, R², R³, R⁴, R^{1'}, R^{2'}, R^{3'}, R^{4'}, falls diese für -(C₁-C₁₂)-Alkyl, -(C₃-C₁₂)-Cycloalkyl,-(C₃-C₁₂)-Heterocycloalkyl, -(C₆-C₂₀)-Aryl oder -(C₃-C₂₀)-Heteroaryl stehen,
jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl,-CONH-(C₁-C₁₂)-Alkyl, -CONH-(C₃-C₁₂)-Cycloalkyl, -CO-(C₁-C₁₂)-Alkyl, -CO-(C3-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl,-(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können.

2. Verfahren nach Anspruch 1,
wobei R¹, R², R³, R⁴, R^{1'}, R^{2'}, R^{3'}, R^{4'}, falls diese für -(C₁-C₁₂)-Alkyl, -(C₃-C₁₂)-Cycloalkyl, -(C₃-C₁₂)-Heterocycloalkyl, -(C₆-C₂₀)-Aryl, oder -(C₃-C₂₀)-Heteroaryl stehen,
jeweils unabhängig voneinander mit einem oder mehreren Substituenten ausgewählt aus -(C₁-C₁₂)-Alkyl, -(C₃-C₁₂)-Cycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl substituiert sein können.

3. Verfahren nach einem der Ansprüche 1 bis 2,
wobei R¹, R², R³, R⁴, R^{1'}, R^{2'}, R^{3'}, R^{4'} jeweils unabhängig voneinander ausgewählt sind aus -(C₁-C₁₂)-Alkyl, -(C₃-C₁₂)-Cycloalkyl, -(C₃-C₁₂)-Heterocycloalkyl, -(C₆-C₂₀)-Aryl, -(C₃-C₂₀)-Heteroaryl;
und mindestens einer der Reste R¹, R², R³, R⁴ bzw. mindestens einer der Reste R^{1'}, R^{2'}, R^{3'}, R^{4'} für einen -(C₃-C₂₀)-Heteroarylrest steht.

4. Verfahren nach einem der Ansprüche 1 bis 3,
wobei mindestens zwei der Reste R¹, R², R³, R⁴ bzw. mindestens zwei der Reste R^{1'}, R^{2'}, R^{3'}, R^{4'} für einen -(C₃-C₂₀)-Heteroarylrest stehen.

5. Verfahren nach Anspruch 4,
wobei die Reste R¹ und R³ bzw. die Reste R^{1'} und R^{3'} jeweils für einen -(C₃-C₂₀)-Heteroarylrest stehen.

6. Verfahren nach Anspruch 5,
wobei die Reste R¹ und R³ bzw. R^{1'} und R^{3'} jeweils für einen -(C₃-C₂₀)-Heteroarylrest stehen;
und die Reste R² und R⁴ bzw. R^{2'} und R^{4'} jeweils unabhängig voneinander ausgewählt sind aus -(C₁-C₁₂)-Alkyl, -(C₃-C₁₂)-Cycloalkyl, -(C₃-C₁₂)-Heterocycloalkyl, -(C₆-C₂₀)-Aryl.

7. Verfahren nach einem der Ansprüche 1 bis 6,
wobei die Reste R¹, R², R³ und R⁴ bzw. R^{1'}, R^{2'}, R^{3'} und R^{4'}, falls sie für einen Heteroarylrest stehen, einen Heteroarylrest mit fünf oder sechs Ringatomen darstellen.

8. Verfahren nach einem der Ansprüche 1 bis 7,
wobei die Reste R¹, R², R³ und R⁴ bzw. R^{1'}, R^{2'}, R^{3'} und R^{4'}, falls sie für einen Heteroarylrest stehen, ausgewählt sind aus 2-Furyl, 2-Thienyl, N-Methyl-2-Pyrrolyl, N-Phenyl-2-Pyrrolyl, N-(2-Methoxyphenyl)-2-Pyrrolyl, 2-Pyrrolyl, N-Methyl-2-Imidazolyl, 2-Imidazolyl, 2-Pyridyl, 2-Pyrimidyl, wobei die genannten Heteroarylreste nicht weiter substituiert sind.

9. Verfahren nach Anspruch 1,
wobei der zweizähnige Phosphinligand ausgewählt ist aus einer Verbindung nach einer der Formeln **(8)** und **(4)**

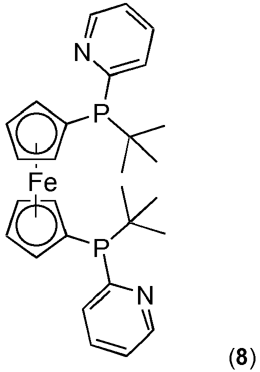

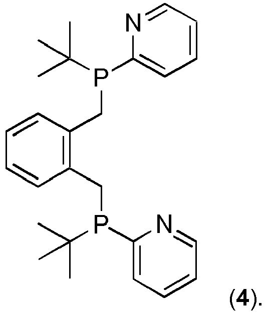

10. Verfahren nach einem der Ansprüche 1 bis 9,
wobei die ethylenisch ungesättigte Verbindung ausgewählt ist aus Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, 1-Octen, 2-Octen, di-n-Buten, oder Mischungen davon.

11. Verfahren nach einem der Ansprüche 1 bis 10,
wobei die Verbindung, welche Pd umfasst, in Verfahrenschritt b) ausgewählt ist aus Palladiumdichlorid, Palladium(II)-acetylacetonat, Palladium(II)-acetat, Dichloro(1,5-cyclooctadiene)palladium(11), Bis(dibenzylideneaceton)palladium, Bis(acetonitrile)dichloropalladium(II), Palladium(cinnamyl)dichlorid.

12. Verfahren nach einem der Ansprüche 1 bis 11,
wobei der Alkohol in Verfahrensschritt c) ausgewählt ist aus Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

13. Verfahren nach einem der Ansprüche 1 bis 12,
wobei dem Reaktionsgemisch weniger als 0,1 mol-%, bezogen auf die Stoffmenge der ethylenisch ungesättigten Verbindung, Brønstedsäuren mit einer Säurestärke von pKs ≤ 5 zugesetzt werden.

## Claims

1. Process comprising the following process steps:
a) introducing an ethylenically unsaturated compound;
b) adding a ligand-metal complex comprising Pd and a bidentate phosphine ligand,
or adding a bidentate phosphine ligand and a compound which comprises Pd;
c) adding an alcohol;
d) supplying CO;
e) heating the reaction mixture, the ethylenically unsaturated compound being reacted to form an ester,
where the reaction mixture is admixed with less than 0.1 mol%, based on the amount of substance of the ethylenically unsaturated compound, of Brønsted acids having an acid strength of pKa ≤ 3, **characterized in that** the phosphine ligand is substituted on at least one phosphorus atom by at least one heteroaryl radical;
wherein the bidentate phosphine ligand is selected from a compound according to one of the formulae (**I**) and (**II**) 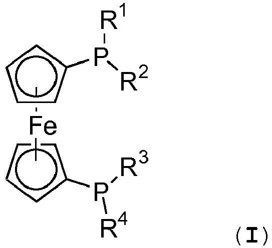 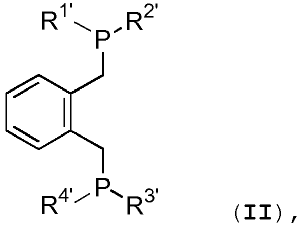 where
R¹, R², R³, R⁴, R^{1'}, R^{2'}, R^{3'}, R^{4'} are each independently selected from -(C₁-C₁₂)-alkyl, -(C₃-C₁₂)-cycloalkyl, -(C₃-C₁₂)-heterocycloalkyl, -(C₆-C₂₀)-aryl, -(C₃-C₂₀)-heteroaryl;
at least one of the R¹, R², R³, R⁴ radicals or at least one of the R^{1'}, R^{2'}, R^{3'}, R^{4'} radicals is a -(C₃-C₂₀)-heteroaryl radical;
and
R¹, R², R³, R⁴, R^{1'}, R^{2'}, R^{3'}, R^{4'}, if they are -(C₁-C₁₂) -alkyl, -(C₃-C₁₂)-cycloalkyl, -(C₃-C₁₂)-heterocycloalkyl, -(C₆-C₂₀)-aryl or -(C₃-C₂₀)-heteroaryl,
may each independently be substituted by one or more substituents selected from
-(C₁-C₁₂)-alkyl, -(C₃-C₁₂)-cycloalkyl, -(C₃-C₁₂)-heterocycloalkyl, -O-(C₁-C₁₂)-alkyl, -O-(C₁-C₁₂)-alkyl- (C₆-C₂₀)-aryl, -O-(C₃-C₁₂)-cycloalkyl, -S-(C₁-C₁₂) -alkyl, -S-(C₃-C₁₂)-cycloalkyl, -COO-(C₁-C₁₂)-alkyl, -COO-(C₃-C₁₂)-cycloalkyl, -CONH- (C₁-C₁₂)-alkyl, -CONH-(C₃-C₁₂)-cycloalkyl, -CO-(C₁-C₁₂)-alkyl, -CO-(C₃-C₁₂) -cycloalkyl, -N-[(C₁-C₁₂)-alkyl]₂, -(C₆-C₂₀)-aryl, -(C₆-C₂)-aryl- (C₁-C₁₂)-alkyl, -(C₆-C₂₀)-aryl-O-(C₁-C₁₂)-alkyl, -(C₃-C₂₀)-heteroaryl, -(C₃-C₂₀)-heteroaryl- (C₁-C₁₂)-alkyl, -(C₃-C₂₀)-heteroaryl-O-(C₁-C₁₂)-alkyl, -COOH, -OH, -SO₃H, -NH₂, halogen.

2. Process according to Claim 1,
where R¹, R², R³, R⁴, R^{1'}, R^{2'}, R^{3'} and R^{4'}, if they are -(C₁-C₁₂)-alkyl, -(C₃-C₁₂)-cycloalkyl, -(C₃-C₁₂)-heterocycloalkyl, -(C₆-C₂₀)-aryl or -(C₃-C₂₀)-heteroaryl,
may each independently be substituted by one or more substituents selected from -(C₁-C₁₂)-alkyl, -(C₃-C₁₂) -cycloalkyl, -O-(C₁-C₁₂)-alkyl, -O-(C₁-C₁₂)-alkyl- (C₆-C₂₀)-aryl, -O-(C₃-C₁₂)-cycloalkyl, -(C₆-C₂₀)-aryl, -(C₆-C₂₀)-aryl- (C₁-C₁₂)-alkyl, -(C₆-C₂₀)-aryl-O-(C₁-C₁₂)-alkyl, -(C₃-C₂₀)-heteroaryl, -(C₃-C₂₀)-heteroaryl- (C₁-C₁₂)-alkyl, -(C₃-C₂₀)-heteroaryl-O-(C₁-C₁₂)-alkyl.

3. Process according to either of Claims 1 and 2,
where R¹, R², R³, R⁴, R^{1'}, R^{2'}, R^{3'} and R^{4'} are each independently selected from -(C₁-C₁₂)-alkyl, -(C₃-C₁₂) -cycloalkyl, -(C₃-C₁₂)-heterocycloalkyl, -(C₆-C₂₀)-aryl, -(C₃-C₂₀)-heteroaryl;
and at least one of the radicals R¹, R², R³ and R⁴, and/or at least one of the radicals R^{1'}, R^{2'}, R^{3'} and R^{4'}, is a -(C₃-C₂₀)-heteroaryl radical.

4. Process according to any of Claims 1 to 3,
where at least two of the R¹, R², R³, R⁴ radicals or at least two of the R^{1'}, R^{2'}, R^{3'}, R^{4'} radicals are a -(C₃-C₂₀)-heteroaryl radical.

5. Process according to Claim 4,
where the R¹ and R³ radicals or the R^{1'} and R^{3'} radicals are each a -(C₃-C₂₀)-heteroaryl radical.

6. Process according to Claim 5,
where the R¹ and R³ or R^{1'} and R^{3'} radicals are each a -(C₃-C₂₀)-heteroaryl radical;
and the R² and R⁴ or R^{2'} or R^{4'} radicals are each independently selected from -(C₁-C₁₂)-alkyl, -(C₃-C₁₂) -cycloalkyl, -(C₃-C₁₂)-heterocycloalkyl, -(C₆-C₂₀)-aryl.

7. Process according to any of Claims 1 to 6,
where the radicals R¹, R², R³ and R⁴ and/or R^{1'}, R^{2'}, R^{3'} and R^{4'}, if they are a heteroaryl radical, are a heteroaryl radical having five or six ring atoms.

8. Process according to any of Claims 1 to 7,
where the radicals R¹, R², R³ and R⁴ and/or R^{1'}, R^{2'}, R^{3'} and R^{4'}, if they are a heteroaryl radical, are selected from 2-furyl, 2-thienyl, N-methyl-2-pyrrolyl, N-phenyl-2-pyrrolyl, N-(2-methoxyphenyl)-2-pyrrolyl, 2-pyrrolyl, N-methyl-2-imidazolyl, 2-imidazolyl, 2-pyridyl, 2-pyrimidyl, the stated heteroaryl radicals not being further substituted.

9. Process according to Claim 1,
where the bidentate phosphine ligand is selected from a compound according to one of the formulae **(8)** and **(4)**

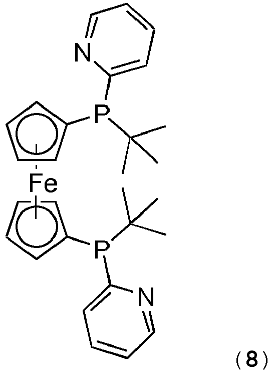

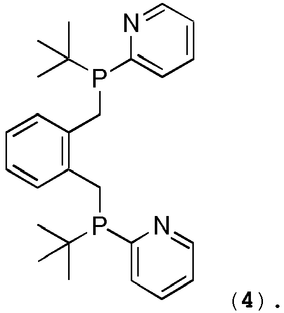

10. Process according to any of Claims 1 to 9,
wherein the ethylenically unsaturated compound is selected from ethene, propene, 1-butene, *cis-* and/or *trans*-2-butene, isobutene, 1,3-butadiene, 1-pentene, *cis-* and/or *trans*-2-pentene, 2-methyl-1-butene, 3-methyl-1-butene, 2-methyl-2-butene, hexene, tetramethylethylene, heptene, 1-octene, 2-octene, di-n-butene, and mixtures thereof.

11. Process according to any of Claims 1 to 10,
wherein the compound comprising Pd in process step b) is selected from palladium dichloride, palladium(II) acetylacetonate, palladium(II) acetate, dichloro(1,5-cyclooctadiene)palladium(II), bis(dibenzylideneacetone)palladium, bis(acetonitrile)dichloropalladium(II), palladium(cinnamyl) dichloride.

12. Process according to any of Claims 1 to 11,
wherein the alcohol in process step c) is selected from methanol, ethanol, 1-propanol, 1-butanol, 1-pentanol, 1-hexanol, 2-propanol, *tert*-butanol, 3-pentanol, cyclohexanol, phenol, and mixtures thereof.

13. Process according to any of Claims 1 to 12,
where the reaction mixture is admixed with less than 0.1 mol%, based on the amount of substance of the ethylenically unsaturated compound, of Brønsted acids having an acid strength of pKa ≤ 5.

## Revendications

1. Procédé comprenant les étapes de procédé :
a) disposition préalable d'un composé éthyléniquement insaturé ;
b) addition d'un complexe ligand-métal comprenant du Pd et d'un ligand bidentate de type phosphine ou addition d'un ligand bidentate de type phosphine et d'un composé qui comprend du Pd ;
c) addition d'un alcool ;
d) introduction de CO ;
e) chauffage du mélange réactionnel, le composé éthyléniquement insaturé étant transformé en ester ;
le mélange réactionnel étant additionné de moins de 0,1% en mole, par rapport à la quantité du composé éthyléniquement insaturé, d'acides de Brönsted présentant une force pKa ≤ 3,
**caractérisé en ce que** le ligand de type phosphine est substitué sur au moins un atome de phosphore par au moins un radical hétéroaryle ;
le ligand bidentate de type phosphine étant choisi parmi un composé selon l'une des formules (I) et (II) 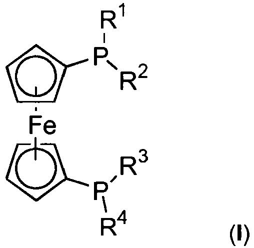 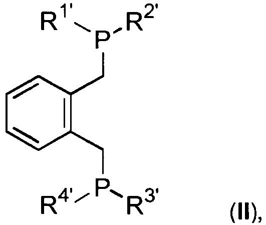 dans lesquelles
R¹, R², R³, R⁴, R^{1'}, R^{2'}, R^{3'}, R^{4'} sont choisis, à chaque fois indépendamment les uns des autres, parmi -(C₁-C₁₂)-alkyle, -(C₃-C₁₂)-cycloalkyle, -(C₃-C₁₂)-hétérocycloalkyle, -(C₆-C₂₀)-aryle, -(C₃-C₂₀)-hétéroaryle ;
au moins un des radicaux R¹, R², R³, R⁴ ou, selon le cas, au moins un des radicaux R^{1'}, R^{2'}, R^{3'}, R^{4'} représente un radical -(C₃-C₂₀)-hétéroaryle ; et
R¹, R², R³, R⁴, R^{1'}, R^{2'}, R^{3'}, R^{4'}, si ceux-ci représentent -(C₁-C₁₂)-alkyle, -(C₃-C₁₂)-cycloalkyle, -(C₃-C₁₂)-hétérocycloalkyle, -(C₆-C₂₀)-aryle ou -(C₃-C₂₀)-hétéroaryle,
peuvent être à chaque fois substitués, indépendamment les uns des autres, par un ou plusieurs substituants choisis parmi -(C₁-C₁₂)-alkyle, -(C₃-C₁₂)-cycloalkyle, -(C₃-C₁₂)-hétérocycloalkyle, -O-(C₁-C₁₂)-alkyle, -O-(C₁-C₁₂)-alkyl-(C₆-C₂₀)-aryle, -O-(C₃-C₁₂)-cycloalkyle, -S-(C₁-C₁₂)-alkyle, -S-(C₃-C₁₂)-cycloalkyle, -COO-(C₁-C₁₂)-alkyle, -COO-(C₃-C₁₂)-cycloalkyle, -CONH- (C₁-C₁₂)-alkyle, -CONH- (C₃-C₁₂)-cycloalkyle, -CO-(C₁-C₁₂)-alkyle, -CO-(C₃-C₁₂)-cycloalkyle, -N-[(C₁-C₁₂)-alkyle]₂, -(C₆-C₂₀)-aryle, -(C₆-C₂₀)-aryl- (C₁-C₁₂)-alkyle, -(C₆-C₂₀)-aryl-O-(C₁-C₁₂)-alkyle, -(C₃-C₂₀)-hétéroaryle, -(C₃-C₂₀)-hétéroaryl-(C₁-C₁₂)-alkyle, -(C₃-C₂₀)-hétéroaryl-O-(C₁-C₁₂)-alkyle, -COOH, -OH, -SO₃H, -NH₂, halogène.

2. Procédé selon la revendication 1,
R¹, R², R³, R⁴, R^{1'}, R^{2'}, R^{3'}, R^{4'}, si ceux-ci représentent -(C₁-C₁₂)-alkyle, -(C₃-C₁₂)-cycloalkyle, -(C₃-C₁₂)-hétérocycloalkyle, -(C₆-C₂₀)-aryle ou -(C₃-C₂₀)-hétéroaryle,
pouvant être substitués, à chaque fois indépendamment les uns des autres, par un ou plusieurs substituants choisis parmi -(C₁-C₁₂)-alkyle, -(C₃-C₁₂)-cycloalkyle, -O-(C₁-C₁₂)-alkyle, -O-(C₁-C₁₂)-alkyl-(C₆-C₂₀)-aryle, -O-(C₃-C₁₂)-cycloalkyle, -(C₆-C₂₀)-aryle, -(C₆-C₂₀)-aryl- (C₁-C₁₂)-alkyle, -(C₆-C₂₀)-aryl-O-(C₁-C₁₂)-alkyle, -(C₃-C₂₀)-hétéroaryle, -(C₃-C₂₀)-hétéroaryl-(C₁-C₁₂)-alkyle, -(C₃-C₂₀)-hétéroaryl-O-(C₁-C₁₂)-alkyle.

3. Procédé selon l'une quelconque des revendications 1 et 2,
R^{1'}, R², R³, R⁴, R^{1'}, R^{2'}, R^{3'}, R^{4'} étant choisis, à chaque fois indépendamment les uns des autres, parmi -(C₁-C₁₂)-alkyle, -(C₃-C₁₂)-cycloalkyle, -(C₃-C₁₂)-hétérocycloalkyle, -(C₆-C₂₀)-aryle, -(C₃-C₂₀)-hétéroaryle ;
et au moins un des radicaux R¹, R², R³, R⁴ ou, selon le cas, au moins un des radicaux R^{1'}, R^{2'}, R^{3'}, R^{4'} représentant un radical -(C₃-C₂₀)-hétéroaryle.

4. Procédé selon l'une quelconque des revendications 1 à 3,
au moins deux des radicaux R¹, R², R³, R⁴ ou, selon le cas, au moins deux des radicaux R^{1'}, R^{2'}, R^{3'}, R^{4'} représentant un radical -(C₃-C₂₀)-hétéroaryle.

5. Procédé selon la revendication 4,
les radicaux R¹ et R³ ou, selon le cas, les radicaux R^{1'} et R^{3'} représentant à chaque fois un radical -(C₃-C₂₀)-hétéroaryle.

6. Procédé selon la revendication 5,
les radicaux R¹ et R³ ou, selon le cas, les radicaux R^{1'} et R^{3'} représentant à chaque fois un radical -(C₃-C₂₀)-hétéroaryle ;
et les radicaux R² et R⁴ ou, selon le cas, R^{2'} et R^{4'} étant choisis, à chaque fois indépendamment l'un de l'autre, parmi -(C₁-C₁₂)-alkyle, -(C₃-C₁₂)-cycloalkyle, -(C₃-C₁₂)-hétérocycloalkyle, -(C₆-C₂₀)-aryle.

7. Procédé selon l'une quelconque des revendications 1 à 6,
les radicaux R¹, R², R³ et R⁴ ou, selon le cas, R^{1'}, R^{2'}, R^{3'} et R^{4'}, s'ils représentent un radical hétéroaryle, représentant un radical hétéroaryle comprenant cinq ou six atomes de cycle.

8. Procédé selon l'une quelconque des revendications 1 à 7,
les radicaux R¹, R², R³ et R⁴ ou, selon le cas, R^{1'}, R^{2'}, R^{3'} et R^{4'}, s'ils représentent un radical hétéroaryle, étant choisis parmi 2-furyle, 2-thiényle, N-méthyl-2-pyrrolyle, N-phényl-2-pyrrolyle, N-(2-méthoxyphényl)-2-pyrrolyle, 2-pyrrolyle, N-méthyl-2-imidazolyle, 2-imidazolyle, 2-pyridyle, 2-pyrimidyle, les radicaux hétéroaryle mentionnés n'étant pas substitués davantage.

9. Procédé selon la revendication 1,
le ligand bidentate de type phosphine étant choisi parmi un composé selon l'une des formules (8) et (4)

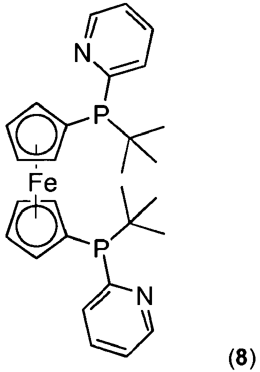

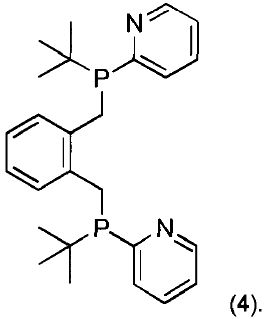

10. Procédé selon l'une quelconque des revendications 1 à 9,
le composé éthyléniquement insaturé étant choisi parmi l'éthylène, le propène, le 1-butène, le cis-2-butène et/ou le trans-2-butène, l'isobutène, le 1,3-butadiène, le 1-pentène, le cis-2-pentène et/ou le trans-2-pentène, le 2-méthyl-1-butène, le 3-méthyl-1-butène, le 2-méthyl-2-butène, l'hexène, le tétraméthyléthylène, l'heptène, le 1-octène, le 2-octène, le di-n-butène ou leurs mélanges.

11. Procédé selon l'une quelconque des revendications 1 à 10,
le composé, comprenant du Pd, dans l'étape b) étant choisi parmi le dichlorure de palladium, l'acétylacétonate de palladium (II), l'acétate de palladium (II), le dichloro(1,5-cyclooctadiène)palladium(II), le bis(dibenzylidène-acétone)palladium, le bis(acétonitrile)dichloropalladium(II), le (cinnamyl)dichlorure de palladium.

12. Procédé selon l'une quelconque des revendications 1 à 11,
l'alcool dans l'étape de procédé c) étant choisi parmi le méthanol, l'éthanol, le 1-propanol, le 1-butanol, le 1-pentanol, le 1-hexanol, le 2-propanol, le tert-butanol, le 3-pentanol, le cyclohexanol, le phénol ou leurs mélanges.

13. Procédé selon l'une quelconque des revendications 1 à 12,
le mélange réactionnel étant additionné de moins de 0,1% en mole, par rapport à la quantité du composé éthyléniquement insaturé, d'acides de Brönsted présentant une force pKa ≤ 5.
